# EUROPEAN PATENT APPLICATION

(11) **EP 1 293 564 A1**
(43) Date of publication of application: **19.03.2003**
(21) Application number: 01936890.1
(22) Date of filing: 07.06.2001
(51) Int. Cl.: C12N 15/09, C12N 15/65, C12N 5/10, C12P 21/02, C12N 15/12

(54) **EXPRESSION VECTOR ENABLING SCREENING OF CELLS WITH HIGH EXPRESSION OF RECOMBINANT PROTEIN, TRANSFORMANT WITH HIGH EXPRESSION OF RECOMBINANT PROTEIN AND UTILIZATION THEREOF**

(30) Priority: 08.06.2000 JP 2000172683; 08.06.2000 JP 2000172684
(71) Applicant: Mitsubishi Pharma Corporation, Osaka-shi, Osaka 541-0046 (JP)
(72) Inventor: SHIBUI, Tatsurou, Mitsubishi Pharma Corporation, Chuo-ku, Tokyo 103-8405 (JP); KITOU, Masahiro, Mitsubishi Pharma Corporation, Chuo-ku, Tokyo 103-8405 (JP); FUKANO, Yasufumi, Mitsubishi Pharma Corporation, Chuo-ku, Tokyo 103-8405 (JP); ITAMI, Seima, Mitsubishi Pharma Corporation, Chuo-ku, Tokyo 103-8405 (JP)
(74) Representative: TER MEER STEINMEISTER & PARTNER GbR
(86) International application number: JP0104801
(87) International publication number: WO01094571

(57) **Abstract**

The present invention relates to an expression vector which comprises an expression unit containing a first selective marker gene and an expression unit containing a second selective marker gene which differs from the first selective marker gene; a transformant having the expression vector; a method for screening cells which express high level of a recombinant protein by use of the transformant; and the like.

The present invention provides a transformant which expresses high level of a target protein, in particular an expression vector which can reproducibly and efficiently screen a highly productive strains of cultured animal cells.

SEQUENCE LISTING

<110> Mitsubishi-Tokyo Pharmaceuticals, Inc.

<120> Expression vector capable of screening cells which express high level of recombinant
protein, transformant which expresses high level of recombinant protein and use thereof

<130> 01017WO0

<150> JP 2000-172683
<151> 2000-06-08
<150> JP 2000-172684
<151> 2000-06-08

<160> 30

<210> 1
<211> 52
<212> DNA
<213> Artificial Sequence

<220>
<223> Primer

<400> 1
tcgaggccat ggtgtcgata acttcgtata gcatacatta tacgaagtta tg 52

<210> 2
<211> 55
<212> DNA
<213> Artificial Sequence

<220>
<223> Primer

<400> 2
gatccataac ttcgtataat gtatgctata cgaagttatc gacaccatgg tggcc 55

<210> 3
<211> 87
<212> DNA
<213> Artificial Sequence

<220>
<223> Primer

<400> 3
ccccgaattc ggatccacta gtcgactaaa taacttcgta tagcatacat tatacgaagt 60
tattgattga acaagatgga ttgcacg 87

<210> 4
<211> 24
<212> DNA
<213> Artificial Sequence

<220>
<223> Primer
<400> 4
ggtcggtcat ttcgaacccc agag 24

<210> 5
<211> 59
<212> DNA
<213> Artificial Sequence

<220>
<223> Primer

<400> 5
ccctgatcag aattcgcagg atccctcgag actagtgatg atcgggccag atatacgcg 59

<210> 6
<211> 51
<212> DNA
<213> Artificial Sequence

<220>
<223> Primer

<400> 6
ccctgatcaa gatctgctag cgtcgactcc ccagcatgcc tgctgctatt g          51

<210> 7
<211> 41
<212> DNA
<213> Artificial Sequence

<220>
<223> Primer

<400> 7
cccaagcttc accatggcct ggattcctct acttctcccc c                     41

<210> 8
<211> 40
<212> DNA
<213> Artificial Sequence

<220>
<223> Primer

<400> 8
cccactagtc tatgaacatt ctgtaggggc cactgtcttc                40

<210> 9
<211> 41
<212> DNA

<213> Artificial Sequence

<220>
<223> Primer

<400> 9
cccaagcttc accatgaagc acctgtggtt cttcctcctg c 41

<210> 10
<211> 35
<212> DNA
<213> Artificial Sequence

<220>
<223> Primer

<400> 10
cccactagtt catttacccg gagacaggga gaggc 35

<210> 11
<211> 30
<212> DNA
<213> Artificial Sequence

<220>
<223> Primer

<400> 11
gctgtacaag taaagcggcc gcgactctag 30

<210> 12
<211> 52
<212> DNA
<213> Artificial Sequence

<220>

<223> Primer

<400> 12
ggactatggt tgctgactaa ttgagttatc agggttattg tctcatgagc gg 52

<210> 13
<211> 25
<212> DNA
<213> Artificial Sequence

<220>
<223> Primer

<400> 13
ctcaattagt cagcaaccat agtcc 25

<210> 14
<211> 36
<212> DNA
<213> Artificial Sequence

<220>
<223> Primer

<400> 14
ccccaagctt ggcctccaaa aaagcctcct cactac 36

<210> 15
<211> 47
<212> DNA
<213> Artificial Sequence

<220>
<223> Primer

<400> 15
ccccaagctt tatccccgct gccatcatgg ttcgaccatt gaactgc 47

<210> 16
<211> 41
<212> DNA
<213> Artificial Sequence

<220>
<223> Primer

<400> 16
ccccagatct aaagccagca aaagacccat gg 32

<210> 17
<211> 41
<212> DNA
<213> Artificial Sequence

<220>
<223> Primer

<400> 17
ccccagatct atcgatcgac tgtgccttct agttgccagc c 41

<210> 18
<211> 40
<212> DNA
<213> Artificial Sequence

<220>
<223> Primer

<400> 18
cccccggtcc gtcagtcctg ctcctcggcc acgaagtgc 40

<210> 19
<211> 33
<212> DNA
<213> Artificial Sequence

<220>
<223> Primer

<400>19
ccccgagctc caccatgtgg gtgaccaaac tcc 33

<210> 20
<211> 32
<212> DNA
<213> Artificial Sequence

<220>
<223> Primer

<400> 20
ccccgagctc ggatcctcat gactgtggta cc 32

<210> 21
<211> 30
<212> DNA
<213> Artificial Sequence

<220>
<223> Primer

<400> 21
gctgtacaag taaagcggcc gcgactctag 30

<210> 22
<211> 52
<212> DNA

<213> Artificial Sequence

<220>
<223> Primer

<400> 22
ggactatggt tgctgactaa ttgagttatc agggttattg tctcatgagc gg 52

<210> 23
<211> 25
<212> DNA
<213> Artificial Sequence

<220>
<223> Primer

<400> 23
ctcaattagt cagcaaccat agtcc 25

<210> 24
<211> 36
<212> DNA
<213> Artificial Sequence

<220>
<223> Primer

<400> 24
ccccaagctt ggcctccaaa aaagcctcct cactac 36

<210> 25
<211> 47
<212> DNA
<213> Artificial Sequence

<220>
<223> Primer

<400> 25
ccccaagctt tatccccgct gccatcatgg ttcgaccatt gaactgc 47

<210> 26
<211> 41
<212> DNA
<213> Artificial Sequence

<220>
<223> Primer

<400> 26
ccccagatct aaagccagca aaagacccat gg 32

<210> 27
<211> 41
<212> DNA
<213> Artificial Sequence

<220>
<223> Primer
<400> 27
ccccagatct atcgatcgac tgtgccttct agttgccagc c 41

<210> 28
<211> 40
<212> DNA
<213> Artificial Sequence

<220>
<223>Primer

<400> 28
cccccggtcc gtcagtcctg ctcctcggcc acgaagtgc 40

<210> 29
<211> 40
<212> DNA
<213> Artificial Sequence

<220>
<223> Primer

<400> 29
ccccgctagc taaataactt cgtatagcat acattatacg aagttataat tgaaaaagcc 60
tgaactcacc gcga 74

<210> 30
<211> 40
<212> DNA
<213> Artificial Sequence

<220>
<223> Primer

<400> 30
cccctattcg gtcgctattc ctttgccctc ggacgagtgc tg 42

## Description

### TECHNICAL FIELD

The present invention relates to an expression vector capable of screening cells which express high level of recombinant protein, a transformant/transfectant which expresses high level of recombinant protein and a method of use thereof. More specifically, the present invention relates to an expression vector capable of screening cells which express high level of expression of industrially useful proteins which can be used as pharmaceuticals and the like, a transformant capable of expressing high level of a target protein by site-specifically introducing a gene of target protein to a highly active transcription site of a host chromosome, and a method for producing the target protein using the transformant.

### BACKGROUND ART

The advances of gene recombination technologies have led to rapid progress, in recent years, on useful protein production technology using gene recombination. For expressing a foreign gene by using gene recombination technology, a suitable host cell and a vector having a foreign gene expression promoter and a selective marker depending on the host cell, are used.

Up to now, numerous researches have been made on microorganisms as host cells for expression, for example, *Escherichia coli* or yeast, which are easy to handle. However, methods using prokaryotes such as *Escherichia coli* are not always effective for all proteins, and the tertiary structures of natural proteins or complicated posttranslational modifications of proteins derived from eukaryotes are not always easily reproduced by such methods.

Namely, in the protein production method using bacteria such as *Escherichia coli* as host, there are some cases wherein proteins of interest may be aggregated in microorganisms, or modifications of sugar chains or the like may not be carried out so that active proteins cannot be produced. Thus, certain limitations to expression of foreign genes have been revealed. Further, animal cells are considered more preferable than microorganisms with respect to expression of drug protein to be administered to humans, and in recent years researches have been vigorously made on expression systems using an animal cell such as cultured cells of higher animals as a host for expression.

Production methods using an animal cell as a host include: one wherein the production is carried out while maintaining an introduced gene extranuclearly; and another wherein a target gene is integrated into a host chromosome. Generally the latter method is employed in order to obtain stable production cells.

In obtaining production strains by introducing the target gene into the host chromosome, the chromosome site on which the gene is to be integrated greatly affects the productivity of the product of the target gene. According to ordinary transformation methods, the gene to be introduced is randomly integrated into a host chromosome, and thus it is necessary to screen a great number of transformant cells in order to obtain strains (highly productive strains) having the gene integrated into sites of high production efficiency (highly active transcriptional site). Such a screening process requires a large amount of labor and time. In particular, in the case where an assay system suitable for assaying highly productive strains is not established, the selection of transformants which express high level of the ultimate target protein requires a particularly large amount of labor and time, and thus rendering this process markedly difficult.

It is an object of the present invention to solve the above-mentioned problems which arise when constructing a system for producing recombinant protein using hosts, particularly animal cells. Thus, the object of the present invention is to provide expression vectors which can reproducibly and efficiently screen transformants which expresses high level of a target protein, in particular, highly productive strains of cultured animal cells.

### DISCLOSURE OF THE INVENTION

The present inventors have conducted intensive researches to solve the above problems. As a result, they have first constructed an expression vector which comprises an expression unit of a drug resistant gene which can be selected in an animal cell and an expression unit of a reporter gene whose expression is easily detectable in an animal cell. Next, the reporter protein expression vector was introduced into a cultured animal cell, and colonies were formed under drug resistant conditions. Then, among the colonies, the colonies which express the reporter gene most efficiently were selected. Marking for site-specifically conducting gene recombination is made in a reporter gene expression unit. A device such as provision of a recombinase recognition sequence is contrived so as to introduce a target genes into a site of strong transcription activity by genetic recombination at any time.

In a clone which expresses the reporter gene most strongly, a reporter gene expression unit is introduced into a site of strong transcription activity. Thus, the reporter gene expression cell can be used as a parent strain in obtaining cells capable of expressing high level of target proteins. Further, using the reporter gene expression cell and introducing various target genes, cells capable of expressing high level of various target proteins can be obtained.

Next, an expression vector was constructed which comprises a non-expression unit containing the drug resistant gene used in the reporter gene expression vector and a drug resistant gene which differs from the reporter gene, and an expression unit containing a gene encoding a target protein. This target protein expression vector was introduced into a reporter gene expression cell, and colonies were formed under drug resistant conditions. A marking (e.g. recombinase recognition sequence) for site-specifically conducting gene recombination is provided in the reporter gene expression unit, and thereby a device is contrived to introduce the target gene into sites of strong transcription activity by site specific gene recombination at any time. Thus, the target protein gene is placed by recombination into a chromosome site of strong transcription activity where the reporter gene has been previously located. Further, only cells wherein the drug resistant gene is changed from the non-expression unit to the expression unit by recombination exhibit drug resistance and form colonies, and therefore the high level of target protein is expressed, and cells capable of expressing high level of the target protein can be easily obtained.

The present invention has been accomplished based on the above findings.

Namely, the present invention provides the following subject matters.
(1) An expression vector which comprises an expression unit containing a first selective marker gene and an expression unit containing a second selective marker gene which differs from the first selective marker gene.
(2) The expression vector according to above (1), wherein the first and second selective marker genes are reporter genes or drug resistant genes.
(3) The expression vector according to above (1) or (2), wherein any one of the first and second selective marker genes is a reporter gene and the other is a drug resistant gene.
(4) The expression vector according to above (2) or (3), wherein the reporter gene is a gene encoding luciferase, alkaline phosphatase, green fluorescence protein or derivatives thereof.
(5) The expression vector according to any of above (2) to (4), wherein the drug resistant gene is a zeocin resistant gene, a kanamycin resistant gene, a chloramphenicol resistant gene, a puromycin resistant gene, an ampicillin resistant gene, a hygromycin resistant gene, a blasticidin resistant gene, a dihydrofolate reductase (dhfr) gene, or a glutamine synthetase gene.
(6) The expression vector according to any of above (1) to (5), which further comprises an expression unit containing a drug resistant gene for gene amplification.
(7) The expression vector according to above (6), wherein the drug resistant gene for gene amplification is a dihydrofolate reductase (dbfr) gene.
(8) The expression vector according to above (6), wherein the drug resistant gene for gene amplification is a glutamine synthetase gene.
(9) The expression vector according to above (6), wherein the expression vector is constructed in such a way that the expression of the drug resistant gene for gene amplification is lowered.
(10) The expression vector according to above (9), wherein the expression vector is constructed in such a way that the expression of the drug resistant gene for gene amplification is lowered by removing an enhancer from a promoter in the expression unit.
(11) The expression vector according to above (9), wherein the expression vector is constructed in such a way that the expression of the drug resistant gene for gene amplification is lowered by removing a poly A addition signal and a transcription termination region in the expression unit.
(12) A transformant having the expression vector of any of above (1) to (11).
(13) The transformant according to above (12), wherein the transformant is an animal cell.
(14) A method for screening a transformant which expresses high level of a target selective marker protein, comprising the steps of:
   introducing the expression vector of any of above (1) to (11) into a host;
   culturing the obtained transformant under conditions suitable for assaying the expression of the first or second selective marker gene contained in the expression vector; and
   selecting the transformant which expresses high level of the target selective marker protein.
(15) A method for screening a transformant which expresses high level of a target selective marker protein, comprising the steps of:
   introducing the expression vector of any of above (6) to (11) into a host;
   culturing the obtained transformant under conditions suitable for assaying the expression of the first or second selective marker gene contained in the expression vector;
   selecting the transformant which expresses high level of the target selective marker gene; and
   culturing the transformant in a medium containing a drug for gene amplification, and selecting the transformant which survives in the medium in which the drug resistant gene for gene amplification is expressed.
(16) The screening method according to above (14) or (15), wherein the first or second selective marker gene is a drug resistant gene, and the assay comprises cultuzzng the transformant in a medium containing the drug and selecting a surviving strains of the transformant.
(17) The screening method according to above (14) or (15), wherein the first or second selective marker gene is a reporter gene, and the assay comprises culturing the transformant in a medium and selecting the surviving Strains of the transformant capable of expressing the reporter gene.
(18) The screening method according to above (17), wherein the assay comprises selecting the surviving strains of the transformant capable of expressing the reporter gene by visually observing and/or determining the intensity of fluorescence or chemiluminescence of the cultured transformants.
(19) The screening method according to above (15) to (18), wherein the drug resistant gene for gene amplification is a dihydrofolate reductase (dhfr) gene and the drug for gene amplification is methotrexate.
(20) The screening method according to any of above (15) to (18), wherein the drug resistant gene for gene amplification is a glutamine synthetase gene and the drug for gene amplification is methionine sulfoximine.
(21) A transformant which expresses high level of a target selective marker protein, which is obtained by the screening method of any of above (14) to (20).
(22) The transformant according to above (21), wherein the transformant is an animal cell.
(23) An expression vector which comprises a non-expression unit containing a third selective marker gene which differs from the first and second selective marker genes contained in the expression vector of above (1) to (11), and an expression unit containing a gene encoding a target protein.
(24) The expression vector according to above (23), wherein the third selective marker gene is a drug resistant gene.
(25) The expression vector according to above (24), wherein the drug resistant gene is a zeocin resistant gene, a kanamycin resistant gene, a chloramphenicol resistant gene, a puromycin resistant gene, an ampicillin resistant gene, a hygromycin resistant gene, a blasticidin resistant gene, a dihydrofolate reductase (dhfr) gene, or a glutamine synthetase gene.
(26) The expression vector according to above (23), wherein the third selective marker gene is a reporter gene.
(27) The expression vector according to above (26), wherein the reporter gene is a gene encoding luciferase, alkaline phosphatase, green fluorescence protein, or derivatives thereof.
(28) A method for screening a transformant which expresses high level of a target protein, comprising the steps of:
   introducing the expression vector of any of above (23) to (27) with a recombinase into the transformant of above (21) or (22) which expresses high level of the target selective marker protein;
   culturing the obtained transformant under conditions suitable for assaying the expression of the third selective marker gene; and
   selecting the transformant which expresses high level of the target protein.
(29) A method for screening a transformant which expresses high level of a target protein, comprising the steps of:
   introducing the expression vector of any of above (23) to (27) with a recombinase into the transformant of above (21) or (22) which expresses high level of the target selective marker protein;
   culturing the obtained transformant under conditions suitable for assaying the expression of the third selective marker gene;
   selecting the transformant which expresses high level of the target protein; and
   culturing the transformant in a medium containing a drug for gene amplification, and selecting the transformant which survives in the medium in which the drug resistant gene for gene amplification is expressed.
(30) The screening method according to above (28) or (29), wherein the first or second selective marker gene contains a recombinase recognition sequence containing an initiation codon, and the non-expression unit containing the third selective marker gene contains a recombinase recognition sequence containing a stop codon.
(31) The screening method according to above (30), wherein the combination of the recombinase recognition sequence and the recombinase is loxp-Cre derived from bacteriophage P1 or FRT-FLP derived from yeast 2 micron DNA.
(32) A transformant which expresses high level of a target protein, which is obtained by the screening method of any of above (28) to (31).
(33) A method for producing a recombinant protein wherein a transformant of above (32) which expresses high level of the target protein is used.
(34) A method for producing a recombinant protein, comprising the steps of:
   culturing the transformant of above (32) which expresses high level of the target protein;
   allowing the transformant to generate and accumulate the recombinant protein in a culture solution; and
   collecting the recombinant protein.
(35) The production method according to (33) or (34), wherein the target protein is an antibody.
(36) The production method according to (33) or (34), wherein the target protein is the hepatocyte growth factor (HGF).

### DETAILED DESCRIPTION OF THE DRAWINGS

Fig. 1 is a schematic view of an expression vector of Example 1 and a transformant having the expression vector.

Fig. 2 is a schematic view of the construction of an expression vector pGS/MLGFP.

Fig. 3 is a schematic view of the construction of an expression vector pCosSLNeo.

Fig. 4 is a schematic view wherein a neomycin resistant gene is changed from a non-expression unit to an expression unit by recombination with Cre recombinase, and a GFP gene is changed to the non-expression unit by recombination.

Fig. 5 is a schematic view of the construction of an expression vector pKS 1.

Fig. 6 is a schematic view of the constructions of an antibody light chain expression vector pEx1-3-1L and an antibody heavy chain expression vector pEx1-3-1H.

Fig. 7 is a schematic view of the construction of an antibody expression vector pEx1-3-1W.

Fig. 8 is a schematic view of the construction of an expression vector pCosSLNeo1-3-1W.

Fig. 9 shows a view of the results of Southern blot hybridization of DNA fragments obtained by cutting genes of individual NSO, M4 and G9 cells with XbaI, and a schematic view of G9 and M4 cells and genes introduced into chromosomes thereof.

Fig. 10 is a schematic view of the construction of an antibody expression vector pMLGFP/DHRR/Zeo.

Fig. 11 is a schematic view of the construction of an HGF expression vector pExHGF.

Fig. 12 is a schematic view of the construction of an expression vector pCosSLNeoHGF.

Fig. 13 is a schematic view of an expression vector of Example 3 and a transformant having the expression vector which expresses a target gene.

Fig. 14 is a schematic view of the construction of an expression vector pMLGFP/DHRR/Zeo.

Fig. 15 is a schematic view of the construction of an expression vector pCosSLHyg.

Fig. 16 is a schematic view wherein a hygromycin resistant gene is changed from a non-expression unit to an expression unit by recombination with a Cre recombinase and a GFP gene is changed to the non-expression unit by recombination.

Fig. 17 is a schematic view of the construction of an expression vector pCosSLHyg1-3-1W.

Fig. 18 shows the results of Southern blot hybridization on DNA fragments obtained by cutting, with HindIII, genes of MKamp 1 cells and MKamp 1 cells wherein 1-3-1 antibody gene was introduced.

### BEST MODE FOR CARRYING OUT THE INVENTION

The selective marker gene to be used in the present invention is a gene which generates an index to distinguish a host wherein the gene is expressed from a host wherein the gene is not expressed. Examples of the selective marker gene include a reporter gene and a drug resistant gene.

Although the type of the reporter gene is not particularly limited, examples thereof include luciferase, alkaline phosphatase, green fluorescence protein (GFP), and genes coding derivatives thereof. The derivatives thereof are proteins comprising one or more amino acid variations (substitution, deletion, and/or addition, and the like) relative to the amino acid sequences of luciferase, alkaline phosphatase, or green fluorescence protein, and these refer to proteins having an equivalent or more physiological activity to that of each naturally occuring protein. For example, in the case of green fluorescence protein, it has been reported that the introduction of a variation into the amino acid sequence of a natural (wild type) GFP enables the obtainment of a derivative thereof having enhanced fluorescence intensity.

A transformant having a green fluorescence protein (GFP) gene exhibits green (fluorescence of 510 nm is emitted under 480 nm excitation light), and therefore it is possible to distinguish a transformant having the GFP gene from a transformant having no GFP gene by identifying the color of the colonies of the transformants. It is further possible to judge the degree of the expression of GFP gene by assaying the color intensity.

Although the drug resistant gene is not particularly limited, examples thereof include a zeocin resistant gene, a kanamycin resistant gene, a chloramphenicol resistant gene, a puromycin resistant gene, an ampicillin resistant gene, a hygromycin resistant gene, a blasticidin resistant gene, a dihydrofolate reductase (dhfr) gene, and a glutamine synfhetase gene.

Transformants having such a drug resistant gene can be selected by culturing the transformants in media containing such a drug.

Although the drug resistant gene for gene amplification is not particularly limited, examples thereof include a dihydrofolate reductase (dhfr) gene and a glutamine synthetase gene. Transformants having the drug resistance gene for gene amplification can be selected by culturing the transformants in a medium containing the drug to which these genes are resistant, and thus the number of copies of the gene which are introduced into host chromosomes can be increased.

In the expression vector of the present invention which comprises an expression unit containing a first selective marker gene, and an expression unit containing a second selective marker gene which differs from the first selective marker gene, it is preferable that any one of the first and second selective marker genes is a reporter gene while the other is a drug resistant gene.

Specifically, it is preferable that the first selective marker gene is a reporter gene and the second selective marker gene is a drug resistant gene, or that the first selective marker gene is a drug resistant gene and the second selective marker gene is a reporter gene.

The "expression unit" in the present invention refers to a unit of a DNA sequence which was constructed so as to express a gene contained in the expression unit. Usually an expression regulatory sequence such as a promoter and/or an enhancer is placed upstream of the gene, and a poly A addition signal sequence and/or a transcriptional termination region or the like are placed downstream of the gene.

The "non-expression unit" in the present invention refers to a unit of a DNA sequence which was constructed so as not to express a gene contained in the non-expression unit. Usually, an expression regulatory sequence comprising a promoter and/or enhancer, etc., and a stop codon is placed upstream of the gene, and a poly A addition signal sequence and/or a transcriptional termination region or the like are placed downstream of the gene.

A preferred promoter to be used in the present invention may be selected depending on a host into which the expression vector is introduced. Examples of promoters include a phage λPL promoter; a T7 promoter; lac, trp, lpp and tac promoters of *E. coli*; an SPO1 promoter and a penP promoter of genus Bacillus; a PHO5 promoter, a PGK promoter, a GAP promoter, an ADH1 promoter, an SUC2 promoter, a GALA promoter and an Mfa promoter of yeast; a polyhedral promoter and a P10 promoter of insect cell; SV40 early and late promoters for animal cells; an LTR promoter of retrovirus; a CMV promoter; an HSV-TK promoter; a metallothionein promoter; 35S promoter for plant cells; and a rice actin gene promoter.

As described below, animal cells are preferably used as hosts to be used in the present invention. Thus, it is preferable to use, as a promoter, SV40 early and late promoters for animal cells, an LTR promoter of retrovirus, a CMV promoter, an HSV-TK promoter, a metallothionein promoter or the like.

Examples of the enhancers include an SV40 enhancer, an adenovirus enhancer, and a cytomegalovirus early enhancer.

In the expression vector of the present invention, if necessary, the signal sequence is placed so as to be added to an N terminal of protein encoded by the gene in each expression unit. Examples of such signal sequences include signal sequences such as PhoA and OmpA for *E. coli* host, SUC2 signal sequence for yeast host, and α -interferon signal sequence for animal cell hosts.

The expression vector of the present invention may contain, as required, a ribosome binding site, a polyadenylation site (SV40 polyadenylation site etc.), a splice donor and acceptor site (DNA sequence derived from SV40 splice site and the like), and transcription termination sequence and 5' non-transcription sequence.

By introducing the expression vector of the present invention into a suitable host, a transformant having the expression vector can be produced.

The type of the host cell is not particularly limited, and examples of the host cells generally include an animal cell, a plant cell, an insect cell, a yeast cell (e.g., *Saccharomyces cerevisae, Schizosaccharomyces pombe*, and *Pichia pastoris*), eukaryotic cells such as genus Aspergillus, and prokaryotic cells such as bacterial cells (e.g., *E. coli, Bacillus subtillis*, Salmonella, Pseudomonas, Streptomyces, and Staphylococcus).

Preferable examples of host cells to be used in the present invention include animal cells, and particularly preferable are mammalian cells. Specific examples of the mammalian cells to be used in the present invention include mouse NSO cells, COS-7 cells, mouse AtT-20 cells, rat GH3 cells, rat MtT cells, mouse MIN6 cells, Vero cells, C127 cells, CHO cells, dhfr gene defective CHO cells, HeLa cells, L cells, BHK cells, BALB3T3 cells, 293 cells, and mouse melanoma cells. Particularly, NSO cells, CHO cells, CHO-DG44 cells, or dhfr gene deficient CHO cells are preferred.

The method for introducing the expression vector of the present invention into a host is not particularly limited, and any known method can be employed. Using, for example, calcium phosphate tranfection, electroporation, lipofection, a method using a gene gun or the like, the expression vector of the present invention can be introduced into the host to prepare a transformant.

The expression vector of the present invention is usually integrated into and maintained in the chromosomes of host as described above.

There may be described two example methods for screening expression cells of the present invention. One method is to screen cells which express high level of selective marker genes, and the other is to screen cells which express high level of target protein genes.

In the method for screening cells which express high level of selective marker genes, the phrase "culturing under conditions suitable for assaying the expression of the first or second selective marker gene" means that, for example, when the selective marker gene is an antibiotic resistant gene, transformants are cultured in a medium containing the corresponding antibiotic and the transformants which are viable under such conditions are selected, thus enabling the obtainment of transformants which express the selective marker gene. Alternatively, when the selective marker gene is a reporter gene, the gene expression is directly monitored, or indirectly monitored, for example, by adding a color substrate, and this enables the obtainment of transformants which express the selective marker gene. For example, in the case where the reporter gene is green fluorescence protein (GFP), cells expressing this gene exhibit green fluorescence. Thus, by visually observing (including e.g. fluorescence microscopic observation) the fluorescence from the cells and/or by measuring fluorescence intensity with a fluorometry apparatus, the degree of the expression of the reporter gene can be assayed.

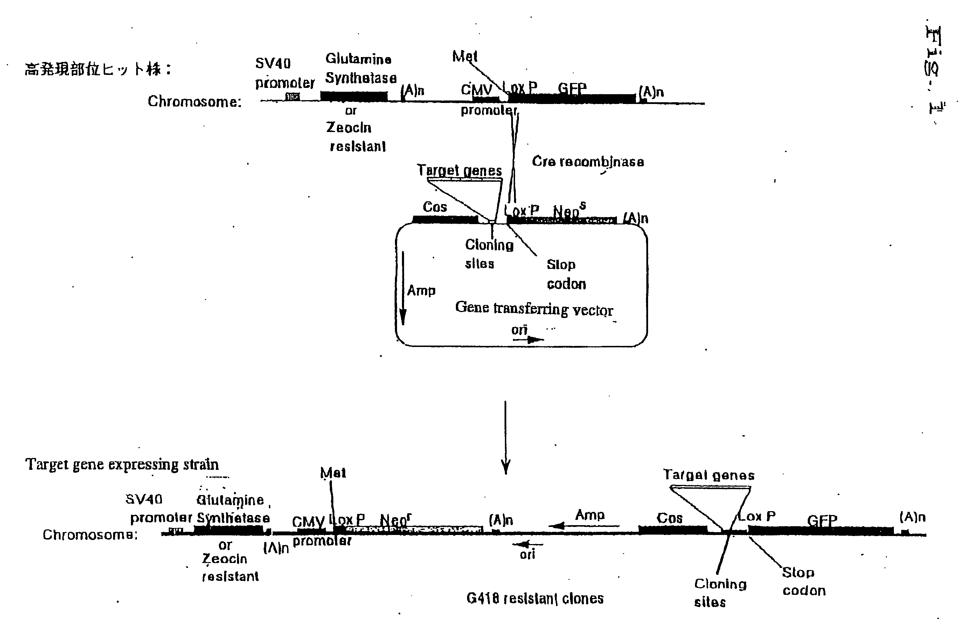

The gene introduced into the reporter gene-expressing transformant which is selected by this assay method is a portion of "the highly expressing site-hits strain" as schematically indicated in Fig. 1.

When the expression vector contains an expression unit which contains a drug resistant gene for gene amplification, the above obtained transformants are further cultured in a medium containing the drug for gene amplification, and the transformants surviving in the medium in which the drug resistant gene for gene amplification is expressed are selected. This process allows for more copies of the gene to be integrated into host chromosomes.

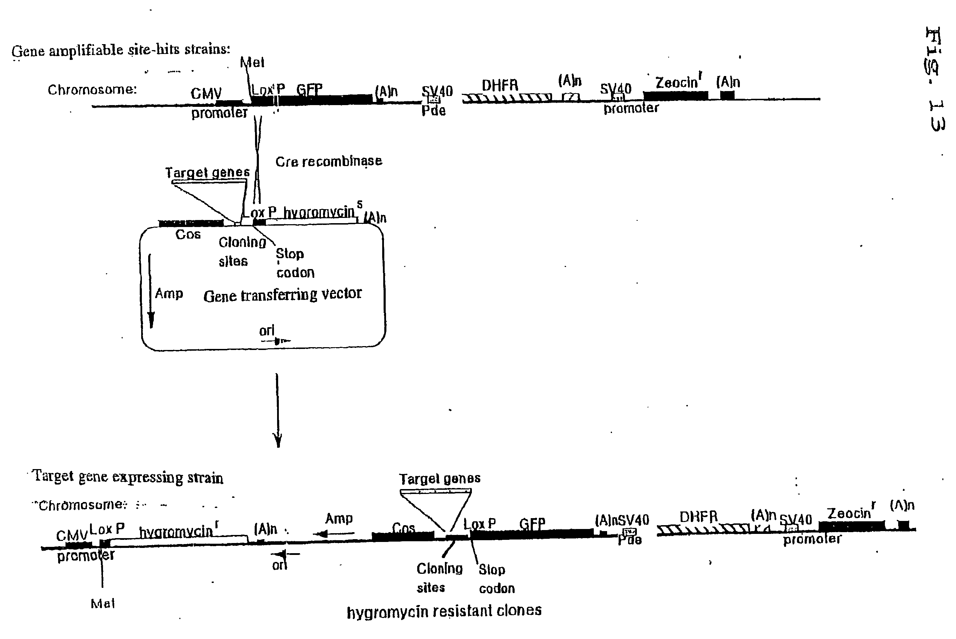

The gene which was introduced into the transformant which is selected by this assay method and expresses the reporter gene and the drug resistance gene for gene amplification, is a portion of "gene amplifiable site-hits strain" as schematically indicated in Fig. 13.

Next, the other method of the present invention for screening an expression cell will be described. In the method for screening high level expression of the target protein gene, the phrase "culturing under conditions suitable for assaying the expression of the third selective marker gene" means that, for example, when the selective marker gene is an antibiotic resistant gene, transformants are cultured in a medium containing the corresponding antibiotic and the transformants which are viable under such conditions are selected, thus enabling the obtainment of transformants which express the selective marker gene. Alternatively, when the selective marker gene is a reporter gene, the gene expression is directly monitored, or indirectly monitored, for example, by adding a color substrate, and this enables the obtainment of transformants which express the selective marker gene. For example, in the case where the reporter gene is green fluorescence protein (GFP), cells which express this gene exhibit green fluorescence. Thus, by visually observing (including e.g. fluorescence microscopic observation) the fluorescence from the cells and/or by measuring fluorescence intensity with a fluorometry apparatus, the degree of the expression of the target protein gene can be assayed.

The gene introduced into the transformant which is selected by this assay method and expresses the gene of the target protein is a portion of "target gene expression strain" as schematically indicated in Fig. 1.

When the expression vector contains an expression unit which contains a drug resistant gene for gene amplification, the following process may be additionally carried out: that is, further culturing the above obtained transformants in a medium containing the drug for gene amplification; and selecting transformants which express the drug resistant gene for gene amplification and survive in the medium. This process allows for more copies of the gene to be integrated into host chromosomes.

The gene introduced into the transformant which is selected by this assay method and expresses the gene of the target protein is a portion of "target gene expression strain" as schematically indicated in Fig. 13.

The present invention also includes a method for producing the target protein using transformants which express high level of the target protein, which are obtained by the method for screening high level expression of the target protein gene as described above.

Namely, the target protein can be produced by using a suitable medium depending on the type of transformant and culturing cells under suitable pH and temperature conditions.

When the transformant is a mammalian cell, MEM medium, DMEM medium, PRMI1640 medium or the like containing approximately 5 to 20 % of fetal bovine serum is generally used for culturing of cells, and the culturing is carried out for approximately 15 to 72 hours at pH of approximately 6 to 8 and temperature of approximately 30 to 40 °C.

The target protein can be collected, as appropriate, from the cell culture by conventional methods depending on the type of transformant.

For example, when the transformant is a mammalian cell, the following methods may be employed: methods utilizing solubility differences such as salting out or solvent precipitation (ammonium sulfate or ethanol precipitation, etc.); methods utilizing molecular weight differences such as dialysis, ultrafiltration, or gel electrophoresis; methods utilizing electric charge differences such as ion exchange chromatography (anion or cation exchange chromatography); methods utilizing specific affinity such as affinity chromatography; methods utilizing hydrophokbicity differences such as hycrophobic interaction chromatography or reverse phase high performance liquid chromatography; and methods utilizing isoelectric points such as isoelectric focusing.

The recombinant protein may or may not be glycosylated. Further, depending on the host, a protein having methionine at its N terminal can be obtained. With respect to the production of the recombinant protein by the above recombinant DNA technique, a method described in MOLECULAR CLONING, A LABORATORY MANUAL, 2nd Ed (Sambrook et al.; Cold Spring Harbor Laboratory Press, Cold Spring Harbor, New York (1989)) may be referred to

In addition, known Western blotting, activity assay or the like may be used as a method for the confirmation of the isolated and purified target protein. Further, the structure of the purified protein can be revealed by amino acid analysis, amino terminal analysis, primary structure analysis, etc.

The type of gene encoding the target protein of the present invention is not particularly limited, and any gene can be selected. Preferable is a gene encoding a protein which is desired to be expressed in animal cells because of the difficulty in expressing and isolating active protein from *Escherichia coli.* Examples of such proteins include a protein which has a complicated structure and is required to have some modification. Proteins which can be used as pharmaceuticals, agonists against receptors existing on cell surfaces, antagonists, lymphokines, or antibodies may be included therein. Preferably, these are antibodies, hepatocyte growth factors (HGF), etc., as described below.

### Examples

The present invention is described further in detail with reference to the following Examples, but the present invention is not particularly limited by these Examples.

### Example 1: High level production system using mouse myeloma cells

### [Construction of search vectors for myeloma cells]

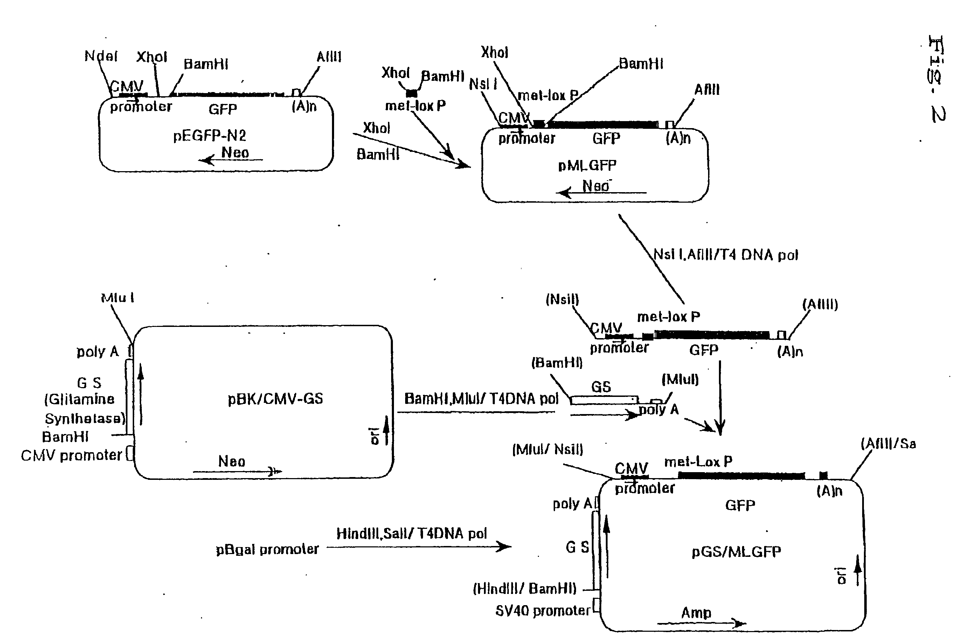

(1) In order to use green fluorescence protein (GFP) as a reporter gene for searching the gene expression intensity, pEGFP-N2 (purchased from Clontech) which was a vector thereof was used. 1 microgram of plasmid DNA thereof was cleaved by reaction with 1 unit of BamHI and XhoI in 50 microliters of system H (50mM tris-HCl, 100mM NaCl, 10mM MgCl₂) at 37°C for 2 hours.
   Approximately 0.5 pmol of marker gene for gene introduction, met-loxp prepared by synthesis, was introduced into the cleaved plasmids using a ligase kit (Takara Shuzo Co., Ltd.). The gene prepared by synthesis had the sequences: 5'TCG AGG CCA TGG TGT CGA TAA CTT CGT ATA GCA TAC ATT ATA CGA AGT TAT G 3' (SEQ ID NO:1); and 5'GAT CCA TAA CTT CGT ATA ATG TAT GCT ATA CGA AGT TAT CGA CAC CAT GGT GGC C 3' (SEQ ID NO:2). After the synthesis using Applied Biosystems Model 394, the gene was prepared by conventional methods. The cleaved plasmids were ligated with the synthesized DNA fragments using a ligase kit (purchased from Takara Shuzo Co., Ltd., the reaction was conducted in accordance with the manual of the kit), and *Escherichia coli* JM109 strain (purchased from Takara Shuzo Co., Ltd., the reaction was conducted in accordance with the attached manual) was transformed, thereby obtaining the desired plasmid pMLGFPNeo. The reporter gene was expressed in the form of a met-loxP-GFP fusion protein by reading through the translation from methionine initiation codon at 9th base from 5' side of the loxP gene (Fig. 2).
   1 microgram of the prepared plasmid DNA was cleaved by reaction with 1 unit of NsiI and AflII in 50 micro of system M at 37°C for 2 hours. Thereafter, dATP, TTP, dGTP, and dCTP (hereinafter referred to as 4NTP) were added to the reaction solution at a final concentration of 2.5 mM, and 1 unit of T4 DNA polymerase was added for reaction at 37°C for 30 minutes so as to blunt the ends.
(2) 1 microgram of glutamine synthetase gene plasmid pBK/CMV-GS (obtained from Dr. Omasa, School of Engineering, Osaka Univ., J. Chem. Eng. Japan, 24(2), pp.184-189 (1998)) was cleaved by reaction with 1 unit of BamHI and Mlul in 50 microliters of system H at 37°C for 2 hours. Thereafter, dATP, TTP, dGTP, and dCTP (hereinafter referred to as 4NTP) were added to the reaction solution at a final concentration of 2.5 mM, and 1 unit of T4 DNA polymerase was added for reaction at 37°C for 30 minutes so as to blunt the ends.
(3) 1 microgram of pBgal-promoter (purchased from Clontech) was cleaved by reaction with 1 unit of HindIII and NdeI in 50 microliters of system M (10mM tris-HCl, 50mM NaCl, 10mM MgCl₂) at 37°C for 2 hours. Thereafter, dATP, TTP, dGTP, and dCTP (hereinafter referred to as 4NTP) were added to the reaction solution at a final concentration of 2.5 mM, and 1 unit of T4 DNA polymerase was added for reaction at 37°C for 30 minutes so as to the blunt ends.

DNA fragments of the above (1), (2), and (3) were ligated with each other by a ligase kit (purchased from Takara Shuzo Co., Ltd., the reaction was conducted in accordance with the manual of the kit), and *Escherichia coli* JM109 strain (purchased from Takara Shuzo Co., Ltd, the reaction was conducted in accordance with the attached manual) was transformed, thereby obtaining the desired plasmid pGS/MLGFP (Fig. 2).

### [Construction of gene transferring vectors]

In order to selectively obtain vectors having site-specifically introduced genes, at first, gene fragments were prepared by synthesizing a gene of stop codon + loxP neomycin fusion protein (stop-loxP-neomycin) by PCR. Primers used for PCR had the following sequences at 5' and 3' sides, respectively: 5'CCC CGA ATT CGG ATC CAC TAG TCG ACT AAA TAA CTT CGT ATA GCA TAC ATT ATA CGA AGT TAT TGA TTG AAC AAG ATG GAT TGC ACG 3' (SEQ ID NO:3); and 5'GGT CGG TCA TTT CGA ACC CCA GAG 3' (SEQ ID NO:4). After the synthesis using Applied Biosystems Model 394, primers were prepared by conventional methods. For PCR reaction, a Perkin Elmer Cetus DNA Thermal Cycler was used, and KOD (purchased from Toyobo Co., Ltd., the reaction conditions were according to the manual) was used as polymerase. Thereafter, desired DNA fragments of approximately 650 bp were purified using 5 % acrylamide electrophoresis according to the method described in Molecular Cloning, Section 6.46-6.48, Cold Spring Harvor. Approximately one microgram of the purified DNA fragments was cleaved by reaction with 1 unit of SpeI and NarI in 50 microliters of system M at 37°C for 2 hours.

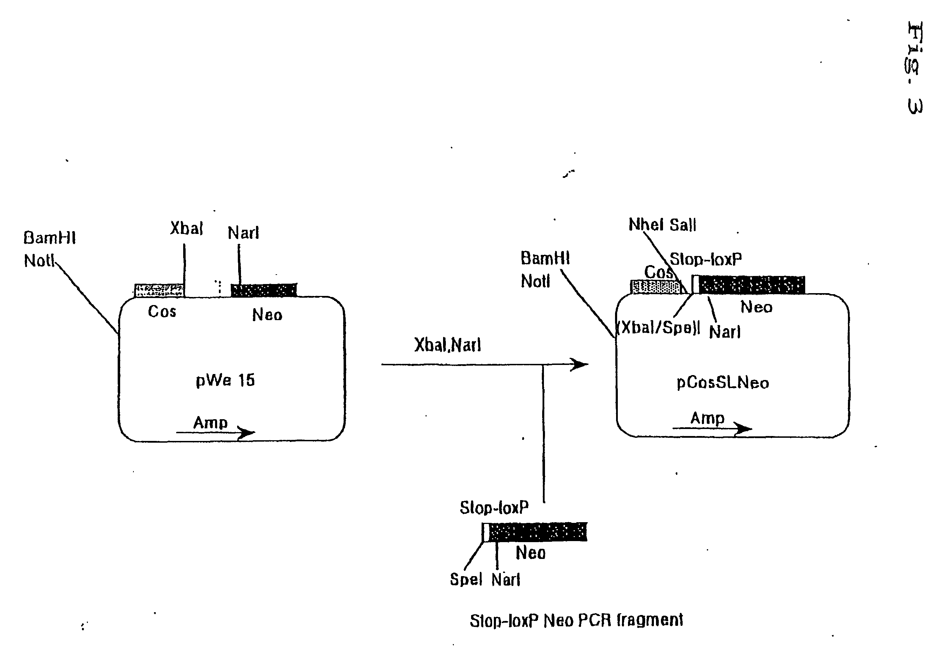

In addition to the above, one microgram of pWe15 DNA (purchased from Stratagene) was cleaved by reaction with 1 unit of XbaI and NarI in 50 microliters of system M ar 37°C for 2 hours. The cleaved DNA fragments and the cleaved plasmids were ligated with each other using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired plasmid pCosSLNeo. The map of plasmid pCosSLNeo is shown in Fig. 3.

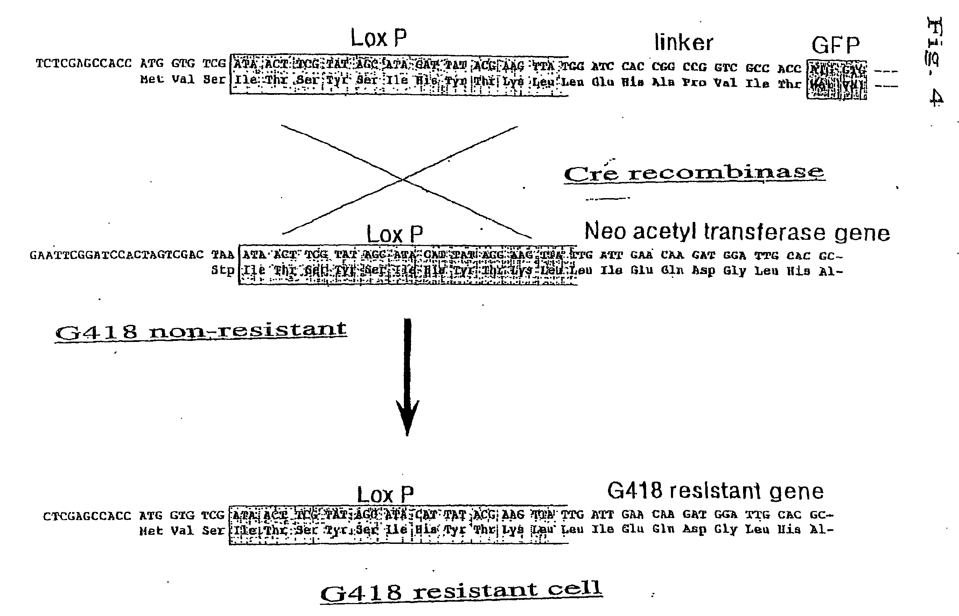

Further, the relationship of the nucleotide sequences of the gene (stop codon loxP) to be site-specifically introduced into a host chromosome and the neomycin resistant gene is shown in Fig. 3. This Stop-loxP-neomycin fusion gene is not usually expressed due to the presence of the stop codon just before the loxP gene. However, only when the fusion gene causes site-specific recombination with met-loxP, a marker gene on the search vector for site-specific gene introduction and the fusion gene is introduced thereinto, met-loxP-neomycin fusion protein was expressed, thereby providing G418 resistance to animal cells (see Fig. 4).

The ligation of the nucleotide between the reporter gene on pGS/MLGFP and the marker gene (met-loxp) on pCosSLNeo for gene introduction is shown in Fig. 4.

### [Incorporation of anticancer antibody gene into gene transferring vector]

### [Preparation of expression vector pKS1]

A region containing CMV promoters of pRC/CMV (purchased from Invitrogen), multi cloning sites, and poly A signal sequence regions (209 - 1250 nucleotide region of gene map of Invitrogen) were amplified by PCR. The primers to be used had the following sequences at 5' and 3' sides: 5'CCC TGA TCA GAA TTC GCA GGA TCC CTC GAG ACT AGT GAT GAT CGG GCC AGA TAT ACG CG 3' (SEQ ID NO:5 and 5'CCC TGA TCA AGA TCT GCT AGC GTC GAC TCC CCA GCA TGC CTG CTG CTA TTG 3' (SEQ ID NO:6), respectvively. After the synthesis using Applied Biosystems Model 394, primers were prepared by conventional methods. For PCR reaction, a Perkin Elmer Cetus DNA Thermal Cycler was used, and KOD (purchased from Toyobo Co., Ltd., the reaction conditions were according to the manual) was used as polymerase.

Thereafter, desired DNA fragment, approximately 1.0 Kbp, was purified using 5% acrylamide electrophoresis according to the method described in Molecular Cloning, Section 6.46-6.48, Cold Spring Harvor, 1998. Approximately 1 microgram of the purified DNA fragment was cleaved by reaction with 1 unit of Bell in 50 microliters of system H at 37°C for 2 hours.

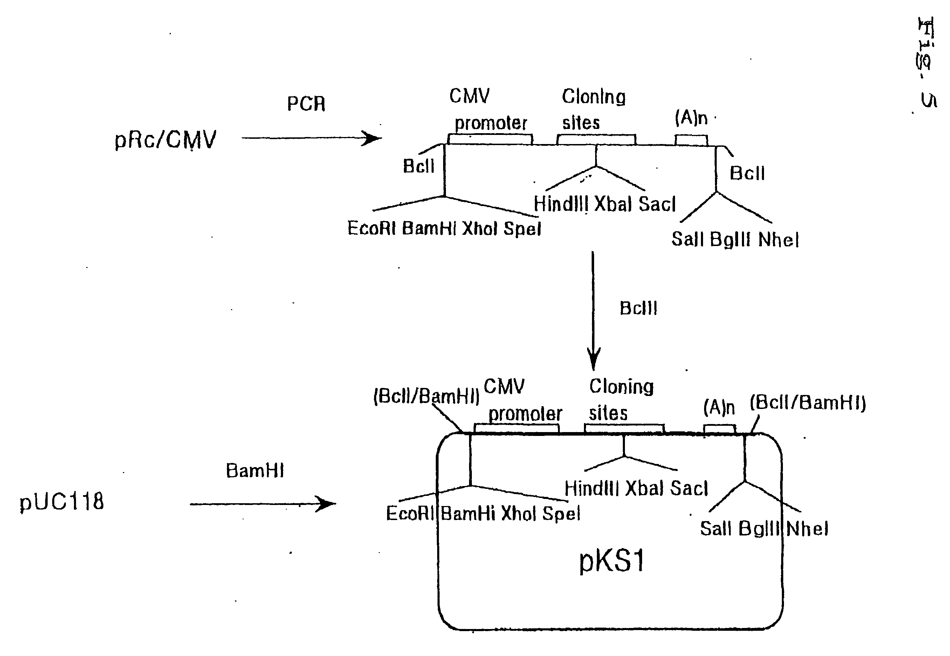

In addition to the above, 1 microgram of pUC118 DNA (purchased from Takara Shuzo Co., Ltd.) was cleaved by reaction with 1 unit of BamHI in 50 microliters of system H at 37°C for 2 hours. The cleaved DNA fragment and the cleaved plasmid were ligated with each other using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired plasmid pKS1 (Fig. 5).

### [Construction of antibody expression genes]

Among 1-3-1 antibody genes described in JP Patent Publication (Unexamined Application) No. 5-304987 (EP520499B1), those of subtype having γ 1 H chain and λ L chain were used, and HindIII and SpeI cleavage sites were added at 5' and 3' sides, respectively, of both chains by PCR. The primers used herein had the following sequences: at 5' side of H chain, 5'CCC AAG CTT CAC CAT GGC CTG GAT TCC TCT ACT TCT CCC CC 3' (SEQ ID NO:7); at 3' side thereof, 5'CCC ACT AGT CTA TGA ACA TTC TGT AGG GGC CAC TGT CTT C 3' (SEQ ID NO:8); at 5' side of L chain, 5'CCC AAG CTT CAC CAT GAA GCA CCT GTG GTT CTT CCT CCT GC 3' (SEQ ID NO.9); and at 3' side thereof, 5'CCC ACT AGT TCA TTT ACC CGG AGA CAG GGA GAG GC 3' (SEQ ID NO:10). After the synthesis using Applied Biosystems Model 394, primers were prepared by conventional methods. For PCR reaction, a Perkin Elmer Cetus DNA Thermal Cycler was used, and KOD (purchased from Toyobo Co., Ltd., the reaction conditions were according to the manual) was used as polymerase.

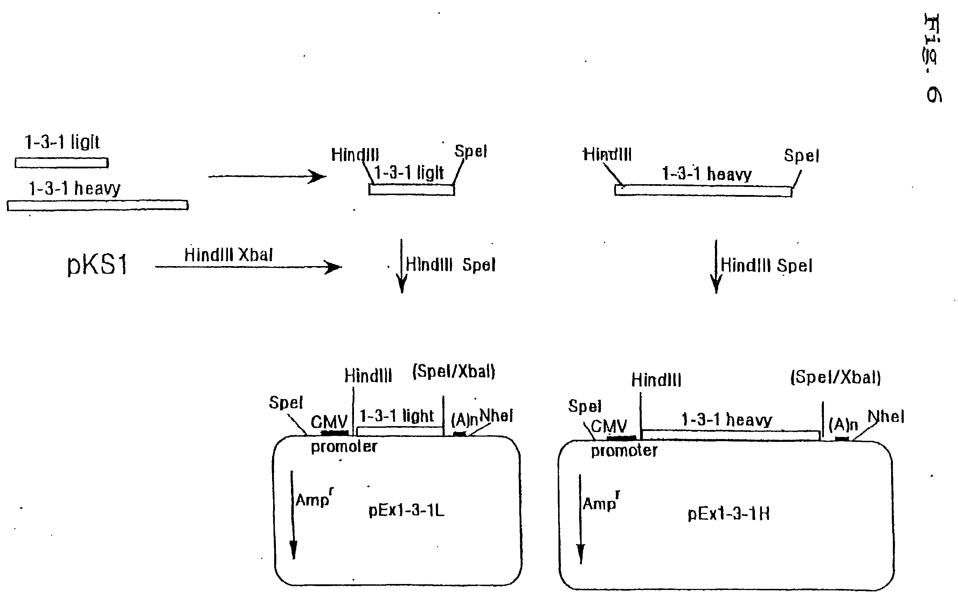

Thereafter, desired DNA fragment was purified using 5 % acrylamide electrophoresis according to the method described in Molecular Cloning, Section 6.46-6.48, Cold Spring Harvor, 1998. Approximately 1 microgram of the purified DNA fragment was cleaved by reaction with 1 unit of HindIII and SpeI in 50 microliters of system M at 37°C for 2 hours. In addition to the above, 1 microgram of the above pKS1 DNA was cleaved by reaction with 1 unit of HindIII and XbaI in 50 microliters of system M at 37°C for'2 hours. The cleaved plasmid was ligated separately with the cleaved H chain DNA fragment and L chain DNA fragment using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining desired H chain expression plasmid pEX1-3-1H and L chain expression plasmid pEX1-3-1L (Fig. 6).

1 microgram of the above pEX1-3-1L DNA was cleaved by reaction with 1 unit of SpeI and NheI in 50 microliters of system M at 37°C for 2 hours. Thereafter, antibody L chain expression DNA fragment of approximately 1.0 Kbp was purified using 5 % acrylamide electrophoresis according to the method described in Molecular Cloning, Section 6.46-6.48, Cold Spring Harvor.

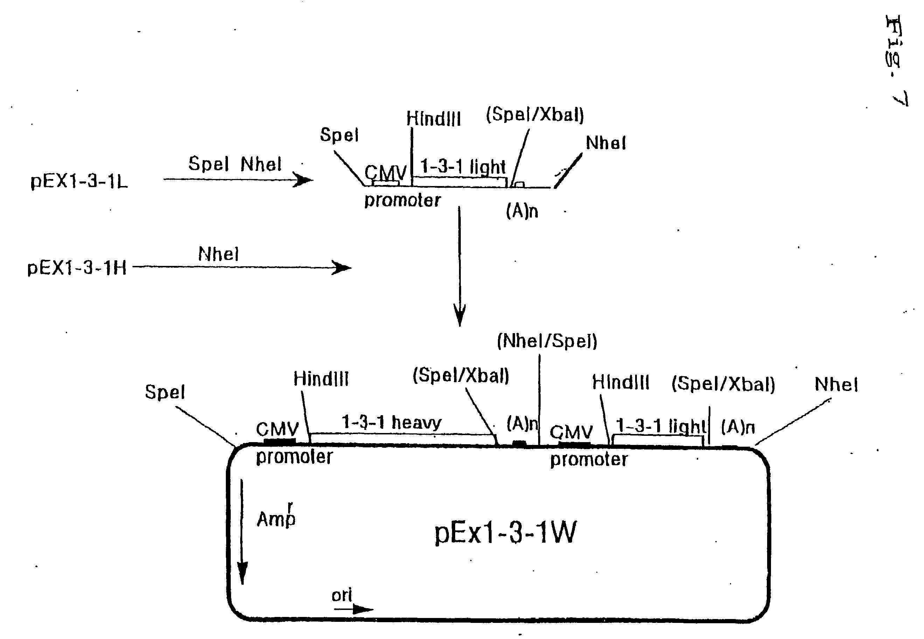

In addition to the above, one microgram of the above pEX1-3-1H DNA was cleaved by reaction with 1 unit of NheI in 50 microliters of system M at 37°C for 2 hours. The cleaved H chain expression plasmid and L chain expression DNA fragment were ligated with each other using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired 1-3-1 antibody expression plasmid pEX1-3-1W (Fig. 7).

### [Preparation of antibody gene site-specifically introduced vectors]

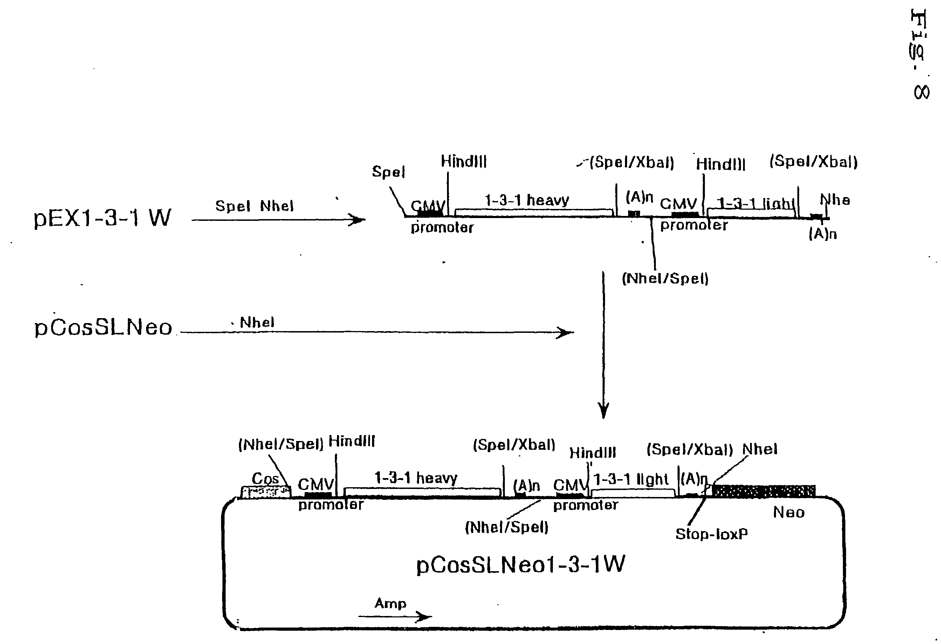

1 microgram of pEX1-3-1W DNA was cleaved by reaction with 1 unit of Spel and NheI in 50 microliters of system M at 37°C for 2 hours. Thereafter, antibody expression DNA fragment, approximately 4.2 Kbp, was purified using 5 % acrylamide electrophoresis according to the method described in Molecular Cloning, Section 6.46-6.48, Cold Spring Harvor. In addition to the above, 1 microgram of the above pCosSLNeo DNA was cleaved by reaction with 1 unit of NheI in 50 microliters of system M at 37°C for 2 hours. The cleaved plasmid was ligated with the cleaved H chain expression plasmids and L chain expression DNA fragments using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired 1-3-1 expression gene site-specifically introduced vector pCosSLNeo1-3-1W (Fig. 8).

### [Screening for high expression site search strain by using search vectors which search for high expression sites in genes for myeloma cells]

### [Preparation of cells used for production]

NSO strain (purchased from The Institute of Physical and Chemical Research) at logarithmic growth phase which was cultured in EX-CELL620 (purchased from JRH BIOSCEENCES) containing 0.4g/L glutamine, was used for transfection, the medium was changed a day before the introduction.

### [Preparation of vectors]

Vectors pGS/MLGFP to be introduced were cleaved with restriction enzyme ScaI (Takara Shuzo Co., Ltd.) which cleaves the vectors at one site irrelevant to the structural gene in the vectors, thereby making them single stranded. 40 microgram of pGS/MLGFP DNA was cleaved by reaction with 40 units of ScaI in 500 microliters of system H at 37°C for 2 hours. After the cleavage, they were subjected to 0.8% agarose gel electrophoresis to verify that they were completely single stranded. Thereafter, the reaction solution was subjected to extraction process with an equal amount of phenol/chloroform solution, and double amount of ethanol was added to an aqueous layer thereof. After the mixture was allowed to stand at -80°C for 1 hour, the precipitates were collected by centrifugation at 15,000 rpm, rinsed with 70% ethanol, vacuum-dried, and dissolved in 100 µL of sterile water.

### [Introduction of vectors into cells]

The genes were introduced into cells by electroporation.

1×10⁷ cells which had a survival rate of greater than 90% were suspended in 0.8 mL of PBS, dispensed into a cuvette together with 100 µL of the prepared vector solution, and mixed gently.

The cuvette was ice-cooled for 5 minutes, and the electrical pulse was applied using Gene Pulser (BIORAD). The application conditions were as follows: 1500 (V), 3µF, and twice applications. After the electrical pulse application, the cuvette was ice-cooled further for 5 minutes.

### [Expression of phenotype of cells in complete medium]

For expression of phenotype of cells, used was IMDM (Gln free) medium (purchased from JRH BIOSCIENCES) added with 0.2 g/L glutamine, 1/20 amount of 20 times concentrated GS supplement (purchased from JRH BIOSCIENCES), and 10 % dialysis FBS (GIBCO).

The electroporated cells were suspended in the above medium, and 50 µL of the suspension having a cell concentration from 4×10⁵ (cell/mL) to 1.6×10³ (cell/mL) was inoculated to each well of a 96-well plate.

### [Selection of recombinant cells]

1 day after the cell inoculation to 96-well, 150 µL of the above medium was added. Thereafter, medium exchange by 100 µL was conducted once every three days using EX-CELL620 medium (purchased from JRH BIOSCIENCES) containing no glutamine until the cells were raised (approximately 3 weeks).

Fluorescence emitted by approximately 300 colonies was visually observed by fluorescence microscopy.

2 strains having medium (M4 strain) and strongest (G9 strain) fluorescence intensity were selected. For the purpose of quantitative determination of fluorescence intensity, the total fluorescence intensity of cells of these strains was measured by FACS analysis.

### [Analysis of fluorescent protein expression strain by FACScan]

For the measurement of the total cell fluorescence intensity, FACS can (BECTON DICKINSON) was employed (air-cooling argon laser, at excitation wavelength of 488 nm). To keep the measurement conditions consistent, all the measurement was carried out with the same sensitivity (PARAMETER: FSC=7.77, SSC=5.0; DETECTOR: FSC=E-1, SSC=300, FL1=504).

As a result, M4 and G9 strains emitted fluorescence of a relative average value of 304 and 1419, respectively.

### [Site-specific introduction of 1-3-1 antibody genes into high expression site search strain with a gene transferring vector]

M4 strain (fluorescence intensity of 304) and G9 strain (fluorescence intensity of 1419) were transfected with pBS185 (site-specific recombinase expression plasmid, purchased from Liferech) and pCosSLNeo1-3-1W using DMRIE-C (purchased from GIBCO-BRL) in accordance with the manual (for each strain, 1.6 microgram of DNA was used for 2 million cells). Using EX-CELL 620 (purchased from JRH), 5 % FBS (purchased from GIBCO-BRL), and 600 mg/l G418 (purchased from SIGMA), they were subjected to selective culturing for 25 days under the conditions of 37°C and 5% CO₂. Then, G418 resistant colonies were selected.

### [Verification of gene introduction site by Southern blotting]

### [Preparation of genome DNA]

From one M4 1-3-1 strain and one G9 1-3-1 strain each of 3-5×10⁶ cells, genome DNAs were extracted using GenomicPrepTM Cells and Tissue DNA Isolation Kit (Pharmacia Biotech) in accordance with the protocols.

### [Southern blotting of genome DNA using GFP probe]

1 microgram of pMLGFP Neo DNA was cleaved by reaction with 1 unit of BamHI and NotI in 50 microliters of system H at 37°C for 2 hours. The desired DNA fragment, approximately 744 bp, was purified using 5 % acrylamide electrophoresis according to the method described in Molecular Cloning, Section 6.46-6.48, Cold Spring Harvor.

Using this DNA fragment, Southern blotting of genome DNA of the transferred strain was conducted. A probe used herein was one which was labeled with fluorescein using a random prime kit (Amersham, Gene Images random prime labelling module) in accordance with the manual. Hybridization and band detection were conducted using Gene Images CDP-Star detection module (Amersham) in accordance with the manual.

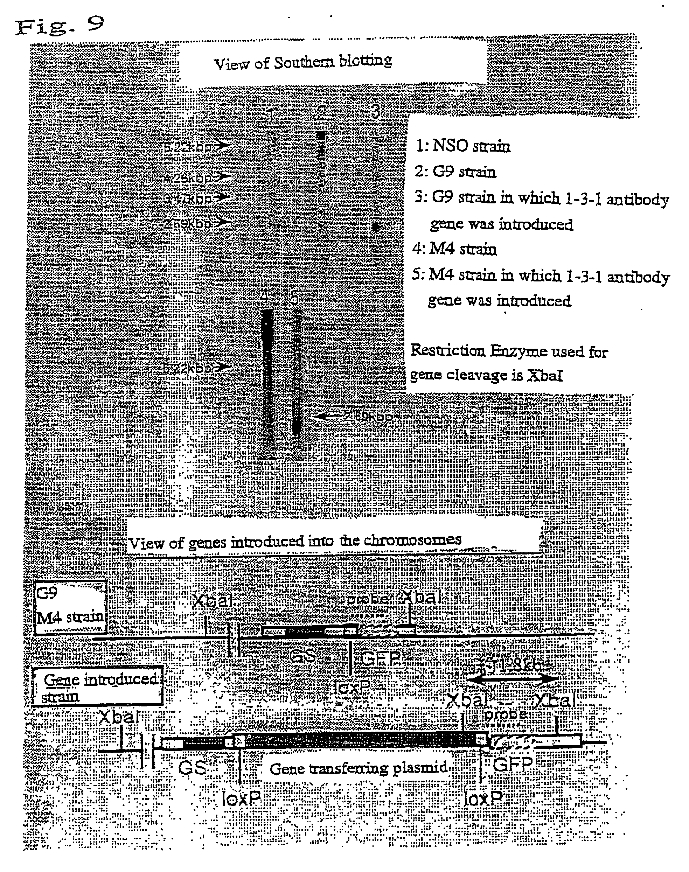

The results of Southern blotting hybridization (Fig. 9) indicate that antibody genes were site-specifically introduced into respective strains which were transfected with 1-3-1 antibody gene site-specific introduction vector and isolated from M4 and G9 strains.

### [Measurement of 1-3-1 antibody production amount using ELISA assay]

50 µl/well of anti-human immuno-globulin goat antibody (purchased from Zymed) solution (diluted to 10 µg/ml with Solution 1) was added into a 96-well plate, and allowed to stand at 37 °C for 2 hours. Thereafter, the solution in each well was discarded, and 250 µl of Solution 2 was added into each well and incubated at 4°C for 16 hours or more. After removing the solution, each well was washed 5 times with Solution 3. Then, antibodies (standard) diluted with Solution 4 or a sample solution (culture supernatant diluted at any given concentration with Solution 4) was added at 50 µl/well and incubated at 37°C for 2 hours. After removing the solution, each well was washed 5 times with Solution 3. Then, Solution 5 was added at 50 µl/well and incubated at 20°C for 40 minutes.

After removing the solution, each well was washed 5 times with PBS, and Solution 9 was added at 100 µl/well and subjected to reaction in the shade at room temperature for approximately 5 minutes. After the equivalent amount of 10 % sulfuric acid was added to stop the reaction, the absorbances A1 and A2 for each well were measured at 11=490nm and 12=650nm using an immuno reader (Inter Med, Immuno Reader NJ-2000), thereby obtaining (A1-A2). The calibration curve was created using diluted standard solutions, and the concentration of 1-3-1 antibodies in the sample was measured.

The composition of each solution was as follows.
Solution 1: PBS containing 0.1 % sodium azide
Solution 2: PBS containing 1 % BSA, and 0.1 % sodium azide
Solution 3: PBS containing 0.05 % Tween 20
Solution 4: PBS containing 1 % BSA
Solution 5: prepared before use by dissolving 2 µl of HRP-labeled anti-human immuno-globulin goat antibody (purchased from Zymed) stock solution in 10 ml of Solution 4
Solution 6: prepared by adding water to 14.2 g of dibasic sodium phosphate up to 500 ml
Solution 7: prepared by adding water to 10.5 g of citric acid monohydrate up to 500 ml
Solution 8: prepared by adding 243 ml of Solution 6 to 257 ml of Solution 5 and adding water up to 1,000 ml
Solution 9: prepared by dissolving 4.0 mg of *o*-phenylenediamine (purchased from Wako Pure Chemical Industries, Ltd.) in 10 ml of Solution 7 and 5 microliters of 30 % (v/v) hydrogen peroxide solution (used within 10 minutes after the preparation)

### [1-3-1 antibody productivity of strains to which 1-3-1 antibody expression genes were site-specifically introduced into the search sites of high expression site search strain]

The following amounts of products were observed by the above ELISA method.

| Parent strain | Fluorescence intensity of parent strain | After introduction of 1-3-1 antibody gene | | |
|---|---|---|---|---|
| | | Fluorescence | G418 resistance | 1-3-1 antibody productivity |
| M4 | 304 | no | resistant | 5mg/l |
| G9 | 1419 | no | resistant | 50mg/l |

### Example 2

### High level production system using Chinese hamster ovary (CHO) cells [construction of search vectors for CHO cells]

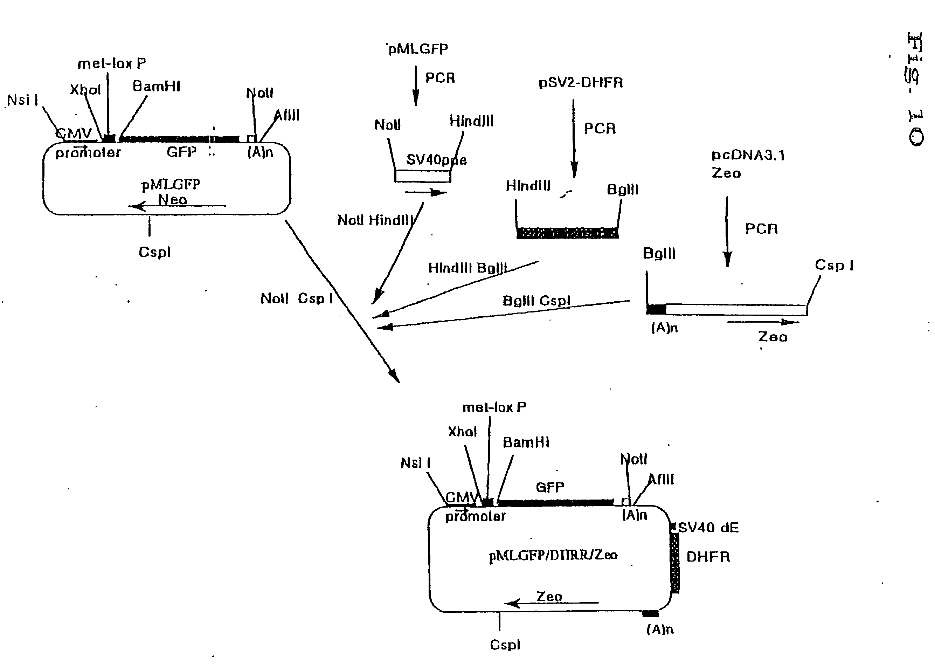

(1) As a reporter gene for searching out gene expression intensity, 1 microgram of plasmid pMLGFP DNA prepared in Example 1 was cleaved by reaction with 1 unit of NotI and CspI in 50 microliters of system M at 37°C for 2 hours.
(2) DNA fragment SV40Pde wherein an enhancer portion (144 base pairs) having a repeating structure of 72 base pairs was removed from SV40 promoter, was amplified and purified by PCR from pMLGFP prepared in Example 1. The primers used for PCR had the following sequences: 
5'GCT GTA CAA GTA AAG CGG CCG CGA CTC TAG3' (SEQ ID NO:11) and
5'GGA CTA TGG TTG CTG ACT AAT TGA GTT ATC AGG GTT ATT GTC TCA
TGA GCG G 3' (SEQ ID NO:12); and
5'CTC AAT TAG TCA GCA ACC ATA GTC C 3' (SEQ ID NO:13) and
 
5'CCC CAA GCT TGG CCT CCA AAA AAG CCT CCT CAC TAC 3' (SEQ ID
NO:14).
 Each amplified fragment was, after purification, further amplified and ligated by PCR using the primers: 
5'GCT GTA CAA GTA AAG CGG CCG CGA CTC TAG3' (SEQ ID NO:11) and
5'CCC CAA GCT TGG CCT CCA AAA AAG CCT CCT CAC TAC 3' (SEQ ID
NO:14).
 The amplification and purification of each PCR fragment were conducted in the same manner as those in Example 1.
   1 microgram of DNA fragment SV40Pde was cleaved by reaction with 1 unit of NotI and HindIII in 50 microliters of system M at 37°C for 2 hours.
(3) Using pMTV-DHFR (Casser, C.S. et al., Proc. Natl. Acad. Sci. USA, 79, 6522-6526(1982)), DHFR (dihydrofolate reductase) gene was amplified by PCR. The primers used for PCR had the following sequences: 
5'CCC CAA GCT TTA TCC CCG CTG CCA TCA TGG TTC GAC CAT TGA ACT GC
3' (SEQ ID NO:15); and
5'CCC CAG ATC TAA AGC CAG CAA AAG ACC CAT GG3' (SEQ ID NO: 16).
 The amplification and purification of PCR fragment were conducted in the same manner as those in Example 1.
   1 microgram of amplified fragment was cleaved by reaction with 1 unit of HindIII and BgIII in 50 microliters of system M (10mM tris-HCl, 50mM NaCl, 2mM MgCl₂) at 37°C for 2 hours.
(4) Using pcDNA3.1 Zeo (purchased from Invitrogen), zeocin resistant gene portion containing BGH poly A addition signal was amplified by PCR. The primers used for PCR had the following sequences: 
5'CCC CAG ATC TAT CGA TCG ACT GTG CCT TCT AGT TGC CAG CC 3' (SEQ
ID NO:17); and
 
5'CCC CCG GTC CGT CAG TCC TGC TCC TCG GCC ACG AAG TGC3 ' (SEQ ID
NO:18).
 The amplification and purification of PCR fragment were conducted in the same manner as those in Example 1.
   1 microgram of amplified fragment was cleaved by reaction with 1 unit of BgIII and CspI in 50 microliters of system H at 37°C for 2 hours.
   DNA fragments of the above (1), (2), (3) and (4) were ligated using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired plasmid pMLGFP/DHFR/Zeo (Fig. 10).

### [Construction of HGF expression gene]

Using HGF gene described in JP Patent Publication (Unexamined Application) No. 3-72883, SacI cleavage sites were added by PCR to both ends of 5' and 3' non translation regions of HGF. The primers used herein had the following sequences: 
at 5' side, 5'CCC CGA GCT CCA CCA TGT GGG TGA CCA AAC TCC 3' (SEQ ID
NO:19); and
at 3' side, 5'CCC CGA GCT CGG ATC CTC ATG ACT GTG GTA CC 3' (SEQ ID
NO:20).
 After the synthesis using Applied Biosystems Model 394, primers were prepared by conventional methods. The PCR reaction and purification of amplified fragments were conducted in the same manner as those in Example 1.

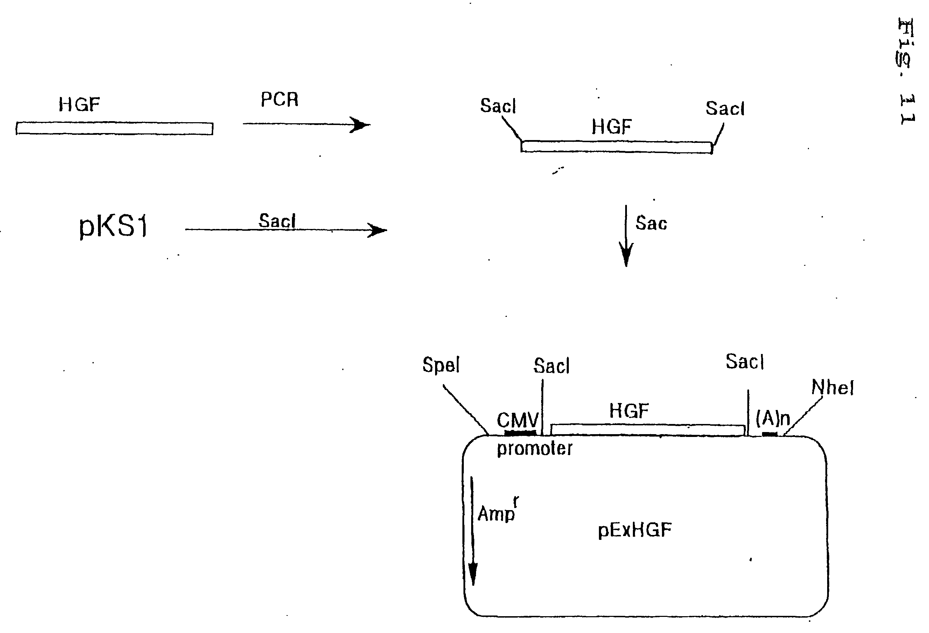

Approximately 1 microgram of purified DNA fragment was cleaved by reaction with 1 unit of SacI in 50 microliters of system L (10mM tris-HCl, 10mM MgCl₂) at 37°C for 2 hours. In addition to the above, 1 microgram of the above pKS1 DNA was cleaved by reaction with 1 unit of SacI in 50 microliters of system L at 37°C for 2 hours. The cleaved plasmid was ligated with the cleaved HGF DNA fragment using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired HGF expression plasmid pEXHGF (Fig. 11).

### [Preparation of site-specifically introduced HGF gene vectors]

1 microgram of the above pEXHGF DNA was cleaved by reaction with 1 unit of SpeI and NheI in 50 microliters of system M at 37°C for 2 hours. Thereafter, HGF expression DNA fragment, approximately 2.2 Kbp, was purified using 5% acrylamide electrophoresis according to the method described in Molecular Cloning, Section 6.46-6.48 (Cold Spring Harvor).

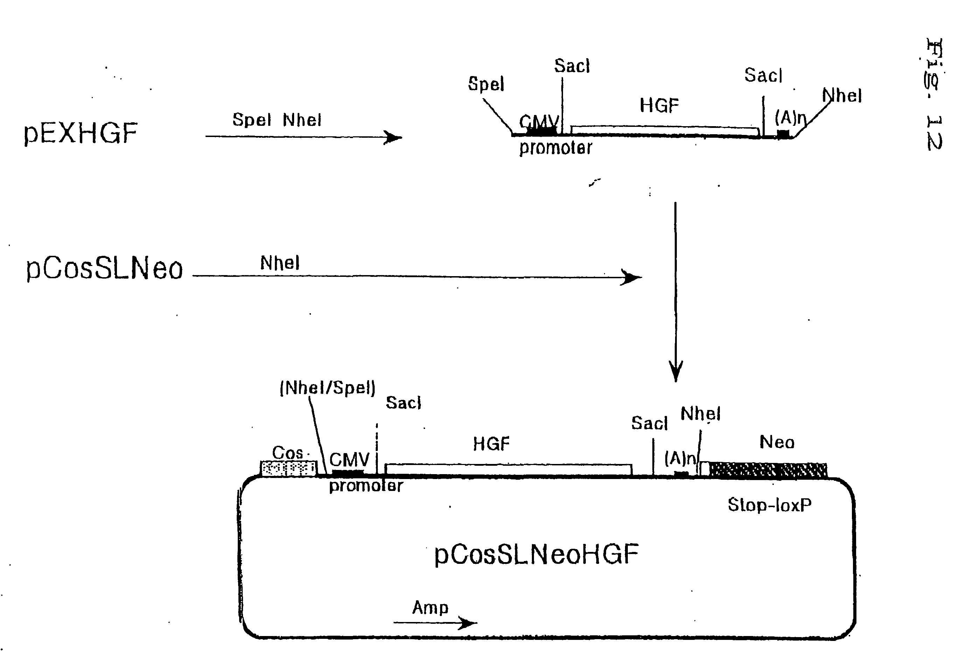

In addition to the above, 1 microgram of pCosSLNeo DNA prepared in Example 1 was cleaved by reaction with 1 unit of NheI in 50 microliters of system M at 37°C for 2 hours. The cleaved plasmid was ligated with the cleaved H chain expression plasmid and L chain expression DNA fragment using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired site-specifically introduced HGF gene expression vectors pCosSLNeoHGF (Fig. 12).

### [Screening for high expression site search strain by using vectors which search for high expression sites in genes for CHO cells]

### [Preparation of cell]

CHODG44 strain at logarithmic growth phase (obtained from Prof. Chaisn, Colombia Univ., Urlaud, G. et al., Proc. Natl. Acad. Sci. USA., 77(1980), 4216-4220) which was cultured in CHO-S-SFM-II medium (purchased from Lifetech) containing 5% FBS (fetal bovine serum: purchased from Lifetech), was used for transfection.

### [Preparation of vector]

Vectors pMLGFP/DHRR/Zeo to be introduced were cleaved with restriction enzyme EcoO109I (purchased from Takara Shuzo Co., Ltd.) which cleaves the vectors at one site irrelevant to structural gene in the vectors, thereby making them single stranded. 40 microgram of pMLGFP Zeo DNA was cleaved by reaction with 40 units of EcoO109 I in 500 microliters of system M at 37°C for 2 hours. After the cleavage, they were verified to be completely single stranded by 0.8% agarose gel electrophoresis. Thereafter, the reaction solution was subjected to extraction process with an equal amount of phenol/chloroform solution. A double amount of ethanol was added to an aqueous layer thereof. Then, the mixture was allowed to stand at -80°C for 1 hour. Thereafter, the precipitates were collected by centrifugation at 15,000 rpm, rinsed with 70% ethanol, vacuum-dried, and dissolved in 100 µL sterile water.

### [Introduction of vector into cell]

The genes were introduced into cells by electroporation.

1×10⁷ cells at logarithmic growth phase were suspended in 0.8 mL PBS, and the suspension was added into a cuvette with 100 µL of the prepared vector solution, and mixed gently.

The cuvette was ice-cooled for 5 minutes, and the electrical pulse was applied using Gene Pulse (BIORAD). The application condition was as follows: 1500 (V), 25µF, and once application. After the electrical pulse application, the cuvette was ice-cooled further for 5 minutes.

The electroporated cells were suspended in the above medium, and 100 µL of the suspension having a cell concentration from 1×10⁵ (cell/mL) to 4×10³ (cell/mL) was inoculated to each well of a 96-well plate.

### [Selection of recombinant cells]

Two days after the inoculation to 96-well, the medium was changed to the above medium added with 200 mg/l zeocin (purchased from Invitrogen). Thereafter, medium change by 100 µL was conducted once every three days until cells were raised (approximately 3 weeks)

Fluorescence emitted by a colony of approximately 1,000 cells was, first, visually observed by fluorescence microscopy. The colony with the strongest intensity (MK strain) was selected.

For the purpose of quantitative determination of fluorescence intensity, the total fluorescence intensity of the cells was measured by FACS analysis in the same manner as Example 1.

The MK strain had a fluorescence intensity of 1419.

### (A) [Site-specific introduction of 1-3-1 antibody expression gene into high expression site search strain by using gene transferring vector]

The above isolated MK strain was transfected with pBS185 (site-specific recombinase expression plasmid, purchased from Lifetech) and pCosSLNeo1-3-1W using Transfectam (Biosepra) in accordance with the manual. Using CHO-S-SFMII medium containing 5% FBS and 400 mg/l G418, the strain was subjected to selective culturing for 25 days under the conditions of 37°C and 5% CO₂. Then, G418 resistant colonies were selected.

### [Measurement of 1-3-1 antibody production amount using EIA assay]

The measurement was conducted in the same manner as described in Example 1.

### [1-3-1 antibody productivity of strains to which 1-3-1 antibody expression genes were site-specifically introduced into the search sites of CHO high expression site search strain]

According to the above EIA method, the following amounts of products were observed.

| Parent strain | fluorescence intensity of parent strain | After introduction of 1-3-1 antibody gene | |
|---|---|---|---|
| | | G418 resistance | 1-3-1 antibody productivity |
| MK strain | 1419 | Resistant | 10 mg/l |

### (B) [Site-specific introduction of HGF expression gene into high expression site search strain by using gene transferring vector]

The above isolated MK strain was transfected with pBS185 (site-specific recombinase expression plasmid, purchased from Lifetech) and pCosSLHGFNeo in the same manner as the above (A). Using CHO-S-SFMII medium containing 5% FBS and 400 mg/l G418, selective culturing was conducted for 25 days under the conditions of 37°C and 5% CO₂. Then, G418 resistant colonies were selected.

### [Measurement of HGF production amount using EIA assay]

50 µl/well of anti HGF monoclonal antibody (obtained by immunizing recombinant HGF to mice) solution (diluted by 100 times with Solution 1) was added into 96-well plate, and allowed to stand at 37°C for 2 hours. Thereafter, the solution in each well was discarded, and 250 µ1 of Solution 2 was added into each well and incubated at 4°C for 16 hours or more. After removing the solution, each well was washed 5 times with Solution 3. Then, HGF diluted with Solution 4 (standard) or a sample solution (culture supernatant diluted with Solution 4 at any given concentration) was added at 50 µl/well and allowed to stand at 4°C for the whole one day. After the solution was removed and each well was washed 5 times with Solution 3, Solution 5 was added at 50 µl/well and the well was incubated at 20°C for 40 minutes. After removing the solution and washing each well 5 times with PBS, 100 µl/well of Solution 9 was added and the reaction was carried out in the shade at room temperature for approximately 5 minutes. After the equivalent amount of 10% sulfuric acid was added to stop the reaction, the absorbances A1 and A2 for each well were measured at 11=490 nm and 12=650 nm using an immuno reader (Inter Med, Immuno Reader NJ-2000), thereby obtaining (A1-A2). The calibration curve was made using diluted standard solutions, and the concentration of HGF in the sample was measured.

The composition of each solution was as follows.
Solution 1: 2% bicarbonate buffer containing 0.1% sodium azide
Solution 2: PBS containing 0.1% gelatin and 0.05% sodium azide
Solution 3: PBS containing 0.05% Tween20
Solution 4: 10 mM phosphate buffer (pH7.5), 0,4M NaCl, 0.1% gelatin, 0.1% CHAPS, 0.05% Tween80
Solution 5: prepared before use by dissolving 2 µl of HRP labeled anti HGF polyclonal antibody stock solution (labeling antibodies obtained by immunizing rabbits with recombinant HGF by means of an HRP labeling kit. The kit was purchased from Amersham and the labeling was conducted in accordance with the direction) into 10 ml of Solution 4
Solution 6: prepared by adding water to 14.2 g of dibasic sodium phosphate up to 500 ml
Solution 7: prepared by adding water to 10.5 g of citric acid monohydrate up to 500 ml
Solution 8: prepared by adding 243 ml of Solution 6 to 257 ml of Solution 5 and adding water up to 1,000 ml
Solution 9: prepared by dissolving 4.0 mg of *o*-phenylenediamine (purchased from Wako Pure Chemical Industries, Ltd.) into 10 ml of Solution 7 and 5 microliters of 30 % (v/v) hydrogen peroxide solution (used within 10 minutes after the preparation)

### [HGF productivity of strain to which HGF expression gene is site-specifically introduced into search sites of high expression site search strain]

According to the above EIA method, the following amount of product was observed.

| Parent strain | Fluorescence intensity of parent strain | After introduction of HGF antibody gene | |
|---|---|---|---|
| | | G418 resistance | HGF antibody productivity |
| MK strain | 1419 | resistan | 0.2 mg/l |

### Example 3

### [Construction of search vector]

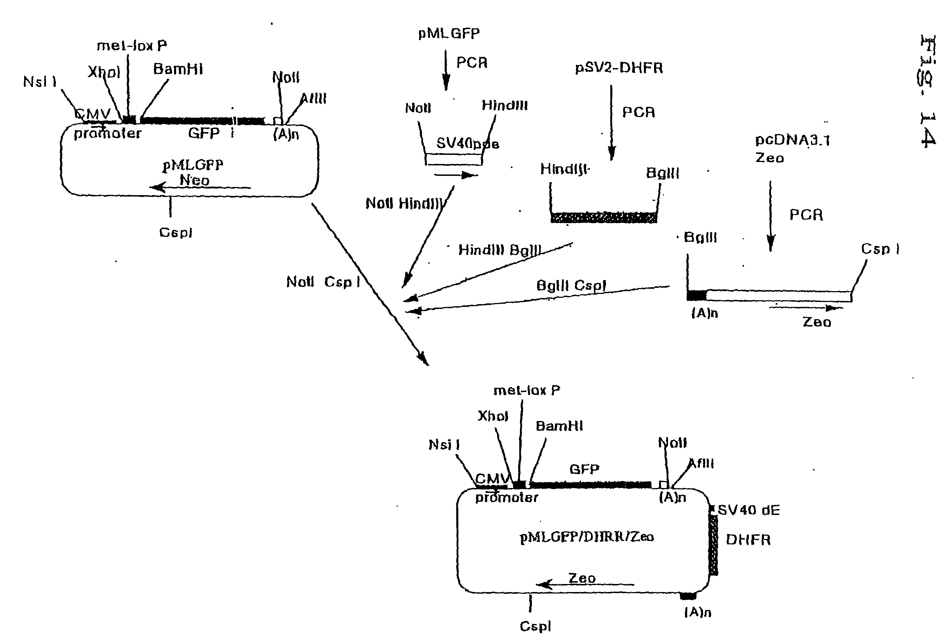

(1) In order to use GFP as a reporter gene, pEGFP-N2 (purchased from Clontech) was used as a vector in the same manner as Example 1. Hereinafter in the same manner as Example 1, *Escherichia coli* JM109 strain (purchased from Takara Sbuzo Co., Ltd., the reaction was conducted in accordance with manual) was transformed, thereby obtaining the desired plasmid pMLGFP. The reporter gene is expressed in the form of met-loxP-GFP fusion protein by reading through the translation from methionine initiation codon at 9th base from 5' side of loxP gene (Fig. 14).
   1 microgram of pMLGFP DNA was cleaved by reaction with 1 unit of NotI and CspI in 50 microliters of system H at 37°C for 2 hours.
(2) DNA fragment SV40Pde wherein an enhancer portion (144 base pairs) having a repeating structure of 72 base pairs was removed from an SV40 promoter, were amplified and purified from pMLGFP by PCR. The primers used for PCR had the following sequences: 
5'GCT GTA CAA GTA AAG CGG CCG CGA CTC TAG3' (SEQ ID NO:21), and
5'GGA CTA TGG TTG CTG ACT AAT TGA GTT ATC AGG GTT ATT GTC TCA
TGA GCG G 3' (SEQ ID NO:22); and
5'CTC AAT TAG TCA GCA ACC ATA GTC C 3' (SEQ ID NO:23), and 5'CCC CAA
GCT TGG CCT CCA AAA AAG CCT CCT CAC TAC 3' (SEQ ID NO:24).
 Each amplified fragment was, after purification, further amplified and ligated by PCR using the primers: 5'GCT GTA CAA GTA AAG CGG CCG CGA CTC TAG3' (SEQ ID NO:21) and 
5'CCC CAA GCT TGG CCT CCA AAA AAG CCT CCT CAC TAC 3' (SEQ ID
NO:24).
 For PCR reaction, a Perkin Elmer Cetus DNA Thermal Cycler was used, and KOD (purchased from Toyobo Co., Ltd., the reaction conditions were in accordance with the manual) was used as polymerase. Thereafter, desired DNA fragment, approximately 750 bp, was purified using 5% acrylamide electrophoresis according to the method described in Molecular Cloning, Section 6.46-6.48, Cold Spring Harvor.
   1 microgram of DNA fragment SV40Pde was cleaved by reaction with one unit of NotI and HindIII in 50 microliters of system M at 37°C for 2 hours.
(3) Using pMTV-DHFR (Casser, C.S. et al., Proc. Natl. Acad. Sci.USA, 79, 6522-6526(1982)), DHFR (dihydrofolate reductase) gene was amplified by PCR. The primers used for PCR were: 
5' CCC CAA GCT TTA TCC CCG CTG CCA TCA TGG TTC GAC CAT TGA ACT
GC 3' (SEQ ID NO:25), and -
5'CCC CAG ATC TAA AGC CAG CAA AAG ACC CAT GG 3' (SEQ ID NO:26).
 For PCR reaction, a Perkin Elmer Cetus DNA Thermal Cycler was used, and KOD (purchased from Toyobo Co., Ltd., the reaction conditions were in accordance with the manual) was used as polymerase. Thereafter, desired DNA fragment, approximately 650 bp, was purified using 5% acrylamide electrophoresis according to the method described in Molecular Cloning, Section 6.46-6.48, Cold Spring Harvor.
   1 microgram of the amplified fragment was cleaved by reaction with 1 unit of HindIII and BglII in 50 microliters of system M (10mM tris-HCl, 50mM NaCl, 10mM MgCl₂) at 37°C for 2 hours.
(4) Using pcDNA3.1 Zeo (purchased from Invitrogen), a Zeocin resistant gene portion containing BGH poy A addition signal was amplified by PCR. The primers used for PCR were: 
5'CCC CAG ATC TAT CGA TCG ACT GTG CCT TCT AGT TGC CAG CC 3' (SEQ
ID NO:27); and
5' CCC CCG GTC CGT CAG TCC TGC TCC TCG GCC ACG AAG TGC 3' (SEQ ID
NO:28).
 For PCR reaction, a Perkin Elmer Cetus DNA Thermal Cycler was used, and KOD (purchased from Toyobo Co., Ltd., the reaction conditions were in accordance with the manual) was used as polymerase. Thereafter, desired DNA fragment, approximately 1.6 Kbp, was purified using 5% acrylamide electrophoresis according to the method described in Molecular Cloning, Section 6.46-6.48, Cold Spring Harvor.
   1 microgram of the amplified fragment was cleaved by reaction with 1 unit of BgIII and CspI in 50 microliters of system H at 37°C for 2 hours.
   DNA fragments of (1), (2), (3) and (4) were ligated with each other using a ligase kit (purchased from Takara Shuzo Co., Ltd., the reaction was conducted in accordance with the direction), and *Escherichia coli* JM109 strain (purchased from Takara Shuzo Co., Ltd., the reaction was conducted in accordance with the manual) was transformed. Then, selection culturing was conducted in LB medium containing 25 mg/l zeocin (purchased from Invitrogen), thereby obtaining the desired plasmid pMLGFP/DHFR/Zeo (Fig. 14).

### [Construction of gene transferring vector]

In order to selectively obtain only vectors having a site-specifically introduced gene, a gene of stop codon+ loxP hygromycin resistant fusion protein (stop-loxP-hygromycin) was amplified and prepared by PCR using hygromycin resistant genes of pIREShyg (purchased from Clontech). Primers used for PCR were: 
at 5' side, 5'CCC CGC TAG CTA AAT AAC TTC GTA TAG CAT ACA TTA TAC
GAA GTT ATA ATT GAA AAA GCC TGA ACT CAC CGC GA 3' (SEQ ID NO:29);
and
at 3' side, 5'CCC CTA TTC GGT CGC TAT TCC TTT GCC CTC GGA CGA GTG
CTG 3' (SEQ ID NO:30).
 The primers were synthesized at SciMedia Ltd and purchased therefrom. For PCR reaction, a Perkin Elmer Cetus DNA Thermal Cycler was used, and KOD (purchased from Toyobo Co., Ltd., the reaction conditions were subjected to the manual) was used as polymerase.

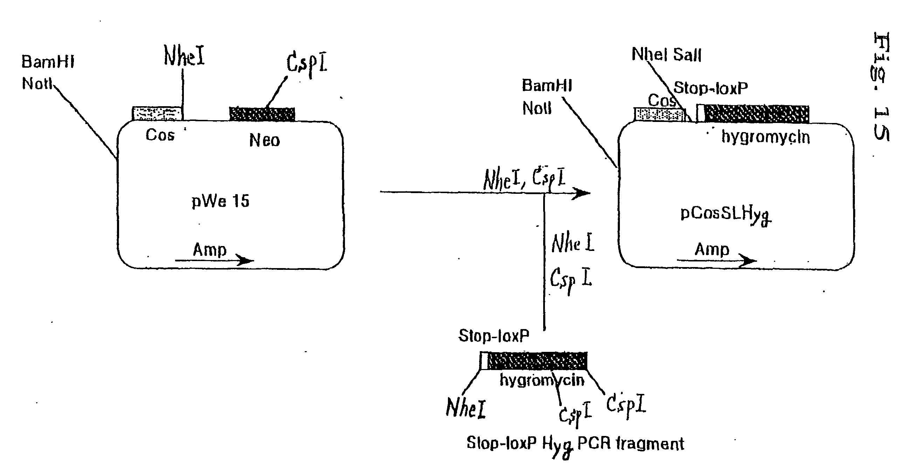

Thereafter, desired DNA fragment, approximately 1,070 bp, was purified using 5% acrylamide electrophoresis according to the method described in Molecular Cloning, Section 6.46-6.48, Cold Spring Harvor. Approximately 1 microgram of the purified DNA fragment was cleaved by reaction with 1 unit of NheI and 0.01 unit of CspI in 50 microliters of system M at 37°C for 2 hours. In addition to the above, 1 microgram of pWe15 DNA was cleaved by reaction with 1 unit of NheI and CspI in 50 microliters of system M at 37°C for 2 hours. The cleaved DNA fragment were ligated with the cleaved plasmid using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired plasmid pCosSLhyg. The map of the plasmid pCosSLhyg is shown in Fig. 15.

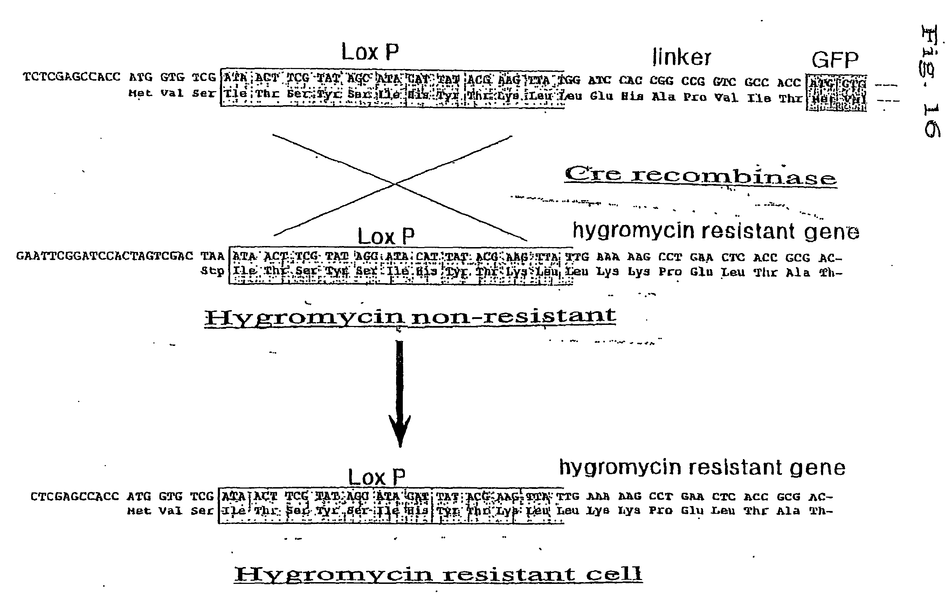

Further, the relationship of the nucleotide sequence between the gene (stop codon loxP) to be site-specifically introduced into a host chromosome and the hygromycin resistant gene is shown in Fig. 14. This Stop-loxP-hygromycin fusion gene is not usually expressed due to the presence of a stop codon just before the loxP gene. However, only when the fusion gene is introduced by site-specific recombination with met-loxP which is a site-specific gene introduction marker gene on the search vector, met-loxP-hygromycin fusion protein is expressed, and thereby hygromycin resistance is provided to animal cells (see Fig. 16).

The ligation of the nucleotide between the reporter gene on pMLGFP/Zeo and the marker gene (met-loxp) on pCosSLhyg for gene introduction is as shown in Fig. 16.

### [Incorporation of anticancer antibody gene into gene transferring vector]

### [Preparation of expression vector pKS1]

The preparation was conducted in the same manner as in Example 1.

### [Construction of antibody expression gene]

The construction was conducted in the same manner as in Example 1.

### [Preparation of antibody gene site-specific introduction vector]

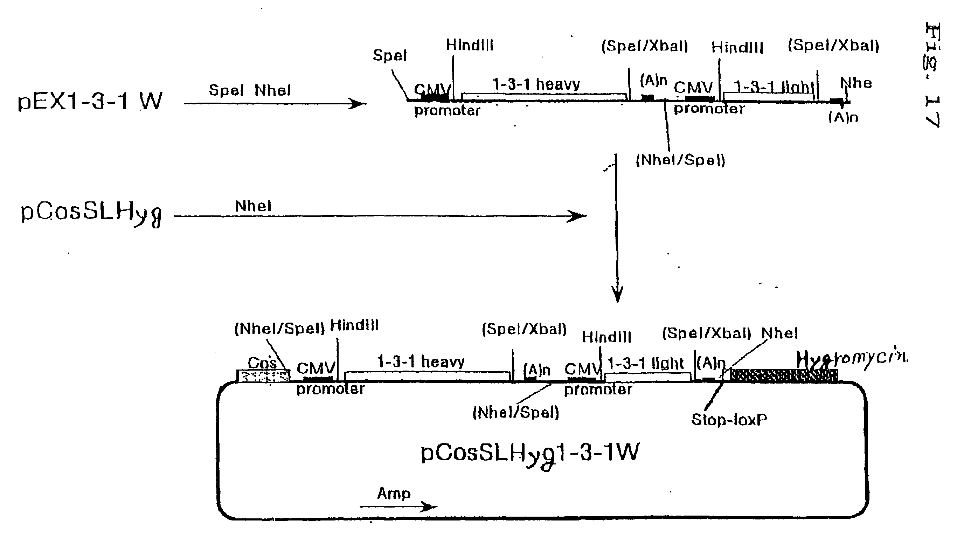

1 microgram of pEX1-3-1W DNA was cleaved by reaction with 1 unit of SpeI and NheI in 50 microliter of system M at 37°C for 2 hours. Thereafter, antibody expression DNA fragment, approximately 4.2 Kbp, was purified using 5% acrylamide electrophoresis according to the method described in Molecular Cloning, Section 6.46-6.48, Cold Spring Harvor. In addition to the above, 1 microgram of the above pCosSLhyg DNA was cleaved by reaction with 1 unit of NheI in 50 microliters of system M at 37°C for 2 hours. The cleaved plasmid was ligated with the cleaved H chain expression plasmid and L chain expression DNA fragments using a ligase kit (purchased from Takara Shuzo Co., Ltd., the reaction was conducted in accordance with the manual), and *Escherichia coli* JM109 strain (purchased from Takara Shuzo Co., Ltd., the reaction was conducted in accordance with the manual) was transformed, thereby obtaining the desired 1-3-1 expression gene site-specific introduction vector pCosSLhyg1-3-1W (Fig. 17).

### [Screening for high expression and gene amplifiable site-hits strains by using search vector]

### [Preparation of cell]

CHODG44 strain at logarithmic growth phase (Chaisn Prof., Columbia Univ., Urlaud, G. et al., Proc. Natl. Acad. Sci. USA., 77(1980), 4216-4220) which was cultured in CHO-S-SFM-II medium (purchased from Lifetech) containing 5% FBS (fetal bovine serum: purchased from Lifetech), was used for transfection, and the medium was changed a day before the introduction.

### [Preparation of vector]

Vectors pMLGFP Zeo to be introduced were cleaved with restriction enzyme EcoO109I (purchased from Takara Shuzo Co., Ltd.) which cleaves the vectors at one site irrelevant to structural gene in the vectors, thereby making them single stranded. 40 micrograms of pMLGFP Zeo DNA was cleaved by reaction with 40 units of EcoO109 I in 500 microliters of system M at 37°C for 2 hours. After the cleavage, they were verified to be completely single stranded by 0.8% agarose gel electrophoresis. Thereafter, the reaction solution was subjected to extraction process with an equal amount of phenol/chloroform solution, and a double amount of ethanol was added to an aqueous layer thereof. After the mixture was allowed to stand at -80°C for 1 hour, the precipitates were collected by centrifugation at 15,000 rpm, rinsed with 70% ethanol, vacuum-dried, and dissolved in 100 µL sterile water.

### [Introduction of vector into cell]

The genes were introduced into cells by electroporation.

1×10⁷ cells at logarithmic growth phase were suspended in 0.8 mL of PBS, added into a cuvette with 100 µL of the prepared vector solution, and mixed gently. The cuvette was ice-cooled for 5 minutes, and the electrical pulse was applied using Gene Pulser (BIORAD). The application conditions were as follows: 1500 (V), 25 µF, and single application. After the electrical pulse application, the cuvette was ice-cooled further for 5 minutes.

The electroporated cells were suspended in the above medium, and 50 µL of the suspension having a cell concentration from 1×10⁵ (cell/mL) to 4×10³ (cell/mL) was inoculated to each well of a 96-well plate.

### [Selection of recombinant cell]

Two days after the cell inoculation to the 96-well, the medium was changed to the above medium added with 200 mg/l zeocin (purchased from Invitrogen). Thereafter, medium changes by 100 µL were conducted once every three days until cells were raised (approximately 3 weeks). Fluorescence emitted by approximately 300 colonies was visually observed at first by fluorescence microscopy. Among these, 16 strains (MKamp candidate strains) having a strong intensity were selected.

### [Analysis of fluorescent protein expression strain by FACScan]

For the measurement of total cell fluorescence intensity, FACScan (BECTON DICKINSON) was employed (air-cooling argon laser, at excitation wavelength of 488 nm). To keep the measurement conditions consistent, all the measurements were carried out with the same sensitivity (PARAMETER: FSC=7.77; DETECTOR: FSC=E-1, FL1=400V). As a result, all of 16 MKamp candidate strains had a relative fluorescence intensity of approximately 100 to 370 on average.

### [Selection of gene amplifiable strain]

MKamp candidate strains were cultured in the above medium, and the cells at logarithmic growth phase were suspended in CHO-S-SFM-II medium (purchased from Lifetech) containing 5% FBS (fetal bovine serum: purchased from Lifetech) and 10nM MTX (methotrexate: purchased from SIGMA), and the suspension was inoculated into a 6-well plate so that each well contained 2×10⁵ cells. Thereafter, the medium was changed by half every three days, and the fluorescence intensity of each colony grown therein was measured by FACS. The measurement results are shown as follows:

| MKamp candidate strain | Fluorescence intensity of original strain | Fluorescence intensity of 5nM MTX resistant colony |
|---|---|---|
| 1 | 259 | 407 |
| 2 | 176 | 382 |
| 3 | 365 | 258 |
| 4 | 194 | 207 |
| 5 | 250 | 232 |
| 6 | 223 | 179 |
| 7 | 277 | 218 |
| 8 | 229 | 142 |
| 9 | 209 | 10 |
| 10 | 155 | 162 |
| 11 | 166 | 187 |
| 12 | 155 | 140 |
| 13 | 274 | 279 |
| 14 | 155 | 140 |
| 15 | 209 | 244 |
| 16 | 184 | 226 |

Among these candidates, 2 strains (underlined) which had remarkably higher fluorescence intensities were selected as gene amplifiable strains, MKamp 1 and MKamp 2.

### [Site-specific introduction of 1-3-1 antibody gene into high expression site search strain by using gene transferring vector]

MKamp 1 strain was transfected with pBS185 (site-specific recombinase expression plasmid, purchased from Lifetech) and pCosSLHyg1-3-1W (for each, 1.0 micrograms of DNA was used for 2 million cells) in accordance with the direction. After the strain was cultured in CHO-S-SFM-II medium (purchased from Lifetech) containing 5% FBS (fetal bovine serum: purchased from Lifetech) for 48 hours under the conditions of 37°C and 5% CO₂, selection culturing was conducted in the above medium containing 200 mg/l hygromycin (purchased from SIGMA) for 25 days under the conditions of 37°C and 5% CO₂, thereby obtaining hygromycin resistant colonies (MKamp1-3-1 strain).

### [Verification of gene introduction site by Southern blotting]

### [Preparation of genome DNA]

From 5×10⁶ cells of the above MKamp1-3-1 strain, genome DNA was extracted using GenomicPrepTM Cells and Tisssue DNA Isolation Kit (Pharmacia Biotech) in accordance with the protocols.

### [Southern blotting of genome DNA using GFP probe]

1 microgram of pMLGFP Neo DNA was cleaved by reaction with 1 unit of BamHI and NotI in 50 microliters of system H at 37°C for 2 hours. Desired DNA fragment, approximately 744 bp, was purified using 5 % acrylamide electrophoresis according to the method described in Molecular Cloning, Section 6.46-6,48, Cold Spring Harvor.

Using this DNA fragment, Southern blotting of genome DNA of the transferred strain was conducted. A probe used herein was one which was labeled with fluorescein using a random prime kit (Amersham, Gene Images random prime labelling module) in accordance with the manual. Hybridization and band detection were conducted using Gene Images CDP-Star detection module (Amersbam) in accordance with the manual.

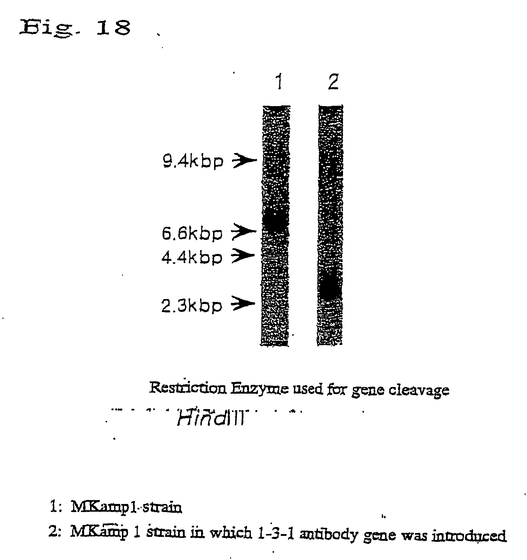

The MKamp strain was transformed with the site-specific gene introduction vector to which 1-3-1 antibody expression gene was introduced. The results of Southern hybridization (Fig. 18) indicate that antibody expression genes were site-specifically introduced into the isolated MKamp1-3-1 strain.

### [Gene amplification of MKamp1-3-1 strain by methotrexate]

MKamp1-3-1 strain was cultured in SFMII medium containing 5% FBS, and the cells at logarithmic growth phase were suspended in the above medium further containing 5nM MTX (methotrexate, purchased from SIGMA). The suspension was inoculated into six 96-well plates so that each well contained 1,000 cells. Thereafter, medium was changed by half with fresh medium every three days, and the 1-3-1 antibody production amount of the growing MTX resistant colony was measured by ELSA assay.

### [Measurement of 1-3-1 antibody production amount using ELISA assay]

The measurement was conducted in the same manner as in Example 1.

### [1-3-1 antibody productivity of MKamp strain to which 1-3-1 antibody expression gene was site-specifically introduced]

The following productivity was observed according to the above ELISA method.

| Parent strain | 1-3-1 antibody gene-introduced strain (before gene amplification) | | Gene-amplified strain by 5nM MTX | |
|---|---|---|---|---|
| | Fluorescence | Hygromycin resistance | 1-3-1 antibody Productivity | 1-3-1 antibody Productivity |
| MKamp 1 | no | resistant | 7 mg/l | 200 mg/l |

### Industrial applicability

By the use of the expression vectors of the present invention, it has become possible to screen cells which express high level of reporter genes, the expression units of which are introduced into the site which shows strong transcription activity. Further, the cell which expresses high level of the reporter gene can be used as a parent strain for producing cells which express high level of various target proteins.

In addition, according to the present invention, after expressing high level of a target protein by site-specifically introducing a target protein gene at a highly transcriptionally active site on a host chromosome, the gene in the chromosome was amplified to obtain a transformant. Therefore, the transformant of the present invention has a high production capability of the target protein.

The present application was filed with claiming priorities based on Japanese Patent Applications Nos.2000-172683 and 2000-172684.

## Claims

1. An expression vector which comprises an expression unit containing a first selective marker gene and an expression unit containing a second selective marker gene which differs from the first selective marker gene.

2. The expression vector according to claim 1, wherein the first and second selective marker genes are reporter genes or drug resistant genes.

3. The expression vector according to claim 1 or 2, wherein any one of the first and second selective marker genes is a reporter gene and the other is a drug resistant gene.

4. The expression vector according to claim 2 or 3, wherein the reporter gene is a gene encoding luciferase, alkaline phosphatase, green fluorescence protein or derivatives thereof.

5. The expression vector according to any of claims 2 to 4, wherein the drug resistant gene is a zeocin resistant gene, a kanamycin resistant gene, a chloramphenicol resistant gene, a puromycin resistant gene, an ampicillin resistant gene, a hygromycin resistant gene, a blasticidin resistant gene, a dihydrofolate reductase (dhfr) gene, or a glucamine synthetase gene.

6. The expression vector according to any of claims 1 to 5, which further comprises an expression unit containing a drug resistant gene for gene amplification.

7. The expression vector according to claim 6, wherein the drug resistant gene for gene amplification is a dihydrofolate reductase (dhfr) gene.

8. The expression vector according to claim 6, wherein the drug resistant gene for gene amplification is a glutamine synthetase gene.

9. The expression vector according to claim 6, wherein the expression vector is constructed in such a way that the expression of the drug resistant gene for gene amplification is lowered.

10. The expression vector according to claim 9, wherein the expression vector is constructed in such a way that the expression of the drug resistant gene for gene amplification is lowered by removing an enhancer from a promoter in the expression unit.

11. The expression vector according to claim 9, wherein the expression vector is constructed in such a way that the expression of the drug resistant gene for gene amplification is lowered by removing a poly A addition signal and a transcription termination region in the expression unit.

12. A transformant having the expression vector of any of claims 1 to 11.

13. The transformant according to claim 12, wherein the transformant is an animal cell.

14. A method for screening a transformant which expresses high level of a target selective marker protein, comprising the steps of:
introducing the expression vector of any of claims 1 to 11 into a host;
culturing the obtained transformant under conditions suitable for assaying the expression of the first or second selective marker gene contained in the expression vector; and
selecting the transformant which expresses high level of the target selective marker protein.

15. A method for screening a transformant which expresses high level of a target selective marker protein, comprising the steps of:
introducing the expression vector of any of claims 6 to 11 into a host;
culturing the obtained transformant under conditions suitable for assaying the expression of the first or second selective marker gene contained in the expression vector;
selecting the trausformant which expresses high level of the target selective marker gene; and
culturing the transformant in a medium containing a drug for gene amplification, and selecting the transformant which survives in the medium in which the drug resistant gene for gene amplification is expressed.

16. The screening method according to claim 14 or 15, wherein the first or second selective marker gene is a drug resistant gene, and the assay comprises culturing the transformant in a medium containing the drug and selecting a surviving strains of the cransformant.

17. The screening method according to claim 14 or 15, wherein the first or second selective marker gene is a reporter gene, and the assay comprises culturing the transformant in a medium and selecting the surviving strains of the transformant capable of expressing the reporter gene.

18. The screening method according to claim 17, wherein the assay comprises selecting the surviving strains of the transformant capable of expressing the reporter gene by visually observing and/or determining the intensity of fluorescence or chemiluminescence of the cultured transformants.

19. The screening method according to any of claims 15 to 18, wherein the drug resistant gene for gene amplification is a dihydrofolate reductase (dhfr) gene and the drug for gene amplification is methotrexate.

20. The screening method according to any of claims 15 to 18, wherein the drug resistant gene for gene amplification is a glutamine synthetase gene and the drug for gene amplification is methionine sulfoximine.

21. A transformant which expresses high level of a target selective marker protein, which is obtained by the screening method of any of claims 14 to 20.

22. The transformant according to claim 21, wherein the transformant is an animal cell.

23. An expression vector which comprises a non-expression unit containing a third selective marker gene which differs from the first and second selective marker genes contained in the expression vector of claims 1 to 11, and an expression unit containing a gene encoding a target protein.

24. The expression vector according to claim 23, wherein the third selective marker gene is a drug resistant gene.

25. The expression vector according to claim 24, wherein the drug resistant gene is a zeocin resistant gene, a kanamycin resistant gene, a chloramphenicol resistant gene, a puromycin resistant gene, an ampicillin resistant gene, a hygromycin resistant gene, a blasticidin resistant gene, a dihydrofolate reductase (dhfr) gene, or a glutamine synthetase gene.

26. The expression vector according to claim 23, wherein the third selective marker gene is a reporter gene.

27. The expression vector according to claim 26, wherein the reporter gene is a gene encoding luciferase, alkaline phosphatase, green fluorescence protein, or derivatives thereof.

28. A method for screening a transformant which expresses high level of a target protein, comprising the steps of:
introducing the expression vector of any of claims 23 to 27 with a recombinase into the transformant of claim 21 or 22 which expresses high level of the target selective marker protein;
culturing the obtained transformant under conditions suitable for assaying the expression of the third selective marker gene; and
selecting the transformant which expresses high level of the target protein.

29. A method for screening a transformant which expresses high level of a target protein, comprising the steps of:
introducing the expression vector of any of claims 23 to 27 with a recombinase into the transformant of claim 21 or 22 which expresses high level of the target selective marker protein;
culturing the obtained transformant under conditions suitable for assaying the expression of the third selective marker gene;
selecting the transformant which expresses high level of the target protein; and
culturing the transformant in a medium containing a drug for gene amplification, and selecting the transformant which survives in the medium in which the drug resistant gene for gene amplification is expressed.

30. The screening method according to claim 28 or 29, wherein the first or second selective marker gene contains a recombinase recognition sequence containing an initiation codon, and the non-expression unit containing the third selective marker gene contains a recombinase recognition sequence containing a stop codon.

31. The screening method according to claim 30, wherein the combination of the recombinase recognition sequence and the recombinase is loxp-Cre derived from bacteriophage P1 or FRT-FLP derived from yeast 2 micron DNA.

32. A trarzsformant which expresses high level of a target protein, which is obtained by the screening method of any of claims 28 to 31.

33. A method for producing a recombinant protein wherein a transformant of claim 32 which expresses high level of the target protein is used.

34. A method for producing a recombinant protein, comprising the steps of:
culturing the transformant of claim 32 which expresses high level of the target protein;
allowing the transformant to generate and accumulate the recombinant protein in a culture solution; and
collecting the recombinant protein.

35. The production method according to claim 33 or 34, wherein the target protein is an antibody.

36. The production method according to claim 33 or 34, wherein the target protein is the hepatocyte growth factor (HGF).
